# EUROPEAN PATENT APPLICATION

(11) **EP 3 978 021 A1**
(43) Date of publication of application: **06.04.2022**
(21) Application number: 20813914.7
(22) Date of filing: 22.05.2020
(51) Int. Cl.: A61K 45/06, A61P 25/04, A61P 29/00, A61P 43/00, C12N 5/10, C12N 15/09, C12N 15/113, A61K 31/7088

(54) **EXPRESSION REGULATOR OF P2X7 RECEPTOR**

(30) Priority: 24.05.2019 JP 2019098049
(71) Applicant: Osaka University, Suita-shi, Osaka 565-0871 (JP)
(72) Inventor: NAGATA, Shigekazu, Suita-shi, Osaka 565-0871 (JP); RYODEN, Yuta, Suita-shi, Osaka 565-0871 (JP); SEGAWA, Katsumori, Suita-shi, Osaka 565-0871 (JP)
(74) Representative: Hoffmann Eitle
(86) International application number: PCT/JP2020/020308
(87) International publication number: WO 2020/241499

(57) **Abstract**

An object is to provide a P2X7 receptor expression modulator. The object is achieved by a P2X7 receptor expression modulator comprising at least one member selected from the group consisting of an Eros (essential for reactive oxygen species) expression modulator and a functional modulator of Eros.

## Description

### Technical Field

The present invention relates to a P2X7 receptor expression modulator and the like.

### Background Art

Extracellular ATP is usually almost nonexistent in body fluids, but its concentration increases in inflammatory sites, cancer microenvironments, immune synapses, and the like. The P2X7 receptor (also referred to as "P2X7" in the present specification), which is known as a ligand-gated cation channel for extracellular ATP, is a plasma membrane localized homotrimeric protein that allows the passage of sodium, calcium, and potassium ions by ATP binding. P2X7 is also known to allow the passage of cations derived from organic compounds with a molecular weight of up to about 900 Da in addition to the above metal ions at the time of ATP binding, and known to induce plasma membrane scrambling, in which membrane phospholipids are transported bidirectionally between the inner and outer leaflets of the plasma membrane, as well as release (shedding) of plasma membrane proteins. Further, P2X7 is known to induce cell death when strongly stimulated and inflammatory response in macrophages, mast cells, and the like (Non-patent Literature (NPL) 1). On the other hand, it has also been reported that P2X7 promotes cell proliferation and is required for the differentiation and maintenance of memory T cells (NPL 2 to NPL 3) .

### Citation List

### Non-patent Literature

NPL 1: Nat Commun 3 1034 (2012)
NPL 2: Nature 559, 264-268 (2018)
NPL 3: FASEB J 23(6), 1685-1693 (2017)

### Summary of Invention

### Technical Problem

An object of the present invention is to provide a P2X7 receptor expression modulator.

### Solution to Problem

As a result of extensive research to achieve the above object, the present inventors found that the expression of the P2X7 receptor can be modulated by using at least one member selected from the group consisting of an Eros (essential for reactive oxygen species) expression modulator and a functional modulator of Eros. The present invention has been accomplished by further conducting research based on this finding. Specifically, the present invention includes the following embodiments.
Item 1. A P2X7 receptor expression modulator comprising at least one member selected from the group consisting of an Eros (essential for reactive oxygen species) expression modulator and a functional modulator of Eros.
Item 2. The modulator according to Item 1, which comprises at least one member selected from the group consisting of an Eros expression suppressor and an Eros function suppressor, and is for suppressing P2X7 receptor expression.
Item 3. The modulator according to Item 2, which comprises an Eros expression suppressor.
Item 4. The modulator according to Item 3, wherein the Eros expression suppressor comprises a polynucleotide.
Item 5. The modulator according to Item 3 or 4, wherein the Eros expression suppressor comprises at least one member selected from the group consisting of an Eros-specific siRNA, an Eros-specific miRNA, an Eros-specific antisense nucleic acid, and an expression cassette thereof, and an Eros gene-editing agent.
Item 6. The modulator according to any one of Items 2 to 5, which is for preventing or ameliorating at least one member selected from the group consisting of inflammation and pain.
Item 7. The modulator according to Item 1, which comprises at least one member selected from the group consisting of an Eros expression promoter and an Eros function promoter, and is for promoting P2X7 receptor expression.
Item 8. The modulator according to Item 7, which comprises an Eros expression promoter.
Item 9. The modulator according to Item 8, wherein the Eros expression promoter comprises an Eros expression cassette.
Item 10. The modulator according to any one of Items 7 to 9, which is for promoting differentiation and/or maintenance of a memory T cell.
Item 11. The modulator according to any one of Items 1 to 10, which is a medicament, a reagent, or a food composition.
Item 12. A T cell into which the modulator according to any one of Items 7 to 10 has been introduced.
Item 13. The T cell according to Item 12, which is capable of modulating Eros expression and/or Eros function so that the Eros expression and/or Eros function is transiently enhanced.
Item 14. The T cell according to Item 12 or 13, into which the modulator according to any one of Items 2 to 5 has been further introduced.
Item 15. The T cell according to Item 14, which is capable of modulating Eros expression and/or Eros function so that the Eros expression and/or Eros function is suppressed, after transiently enhancing the Eros expression and/or Eros function.
   The present invention also includes the following embodiments.
Item 16. A method for modulating P2X7 receptor expression, comprising administering to an animal at least one member selected from the group consisting of an Eros expression modulator and a functional modulator of Eros.
Item 17. A composition for use in modulating P2X7 receptor expression in an animal body, the composition comprising at least one member selected from the group consisting of an Eros expression modulator and a functional modulator of Eros.
Item 18. Use of at least one member selected from the group consisting of an Eros expression modulator and a functional modulator of Eros for the production of a P2X7 receptor expression modulator.
Item 19. A method for modulating P2X7 receptor expression in vitro, comprising contacting a cell with at least one member selected from the group consisting of an Eros expression modulator and a functional modulator of Eros.
Item 20. A method for inhibiting IL-1β, comprising suppressing Eros expression and/or Eros function.

### Advantageous Effects of Invention

The present invention provides a P2X7 receptor expression modulator.

### Brief Description of Drawings

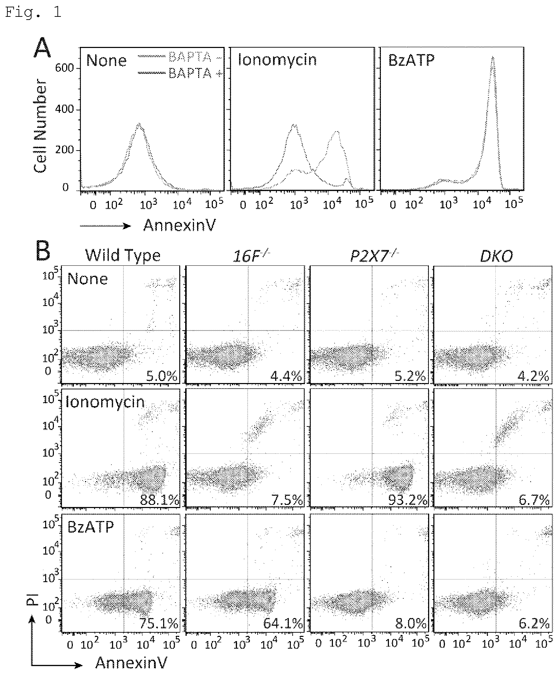

Fig. 1 shows the results of investigation of P2X7-mediated, TMEM16F-independent PtdSer exposure in WR19L cells. (A) Ca²⁺ is not required for Bz-ATP-induced PtdSer exposure in WR19L cells. (B) TMEM16F-dependent or P2X7-dependent PtdSer exposure in WR19L cells.

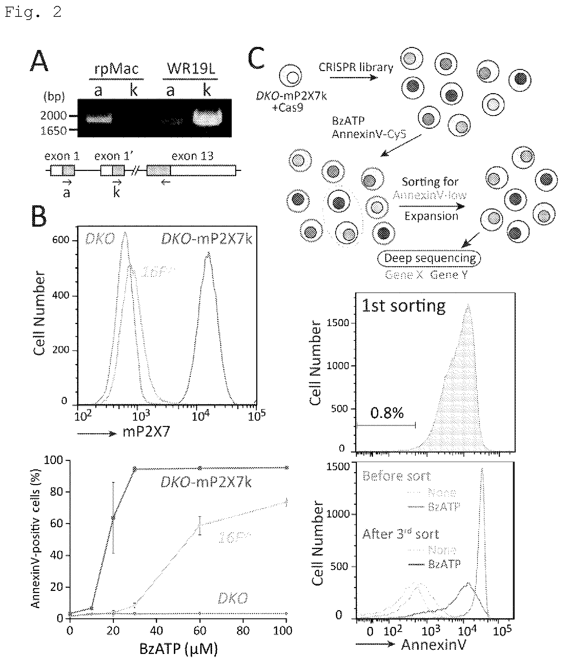

Fig. 2 shows the results of genome-wide CRISPR screening for genes that support P2X7-mediated PtdSer exposure. (A) Predominant expression of the P2X7k isoform in WR19L cells. In the lower panel, the structure of the mP2X7 gene is schematically shown with the positions of primers. (B) mP2X7k-mediated PtdSer exposure. Staining profiles in a SYTOX Blue-negative population are shown. (C) Results of identification of genes that support mP2X7-mediated PtdSer exposure. The upper panel shows a CRISPR screening procedure. The middle panel shows the FACS profile of the first sorting, in which a population sorted for the next step is indicated. The bottom panel shows annexin V staining profiles in a PI-negative population.

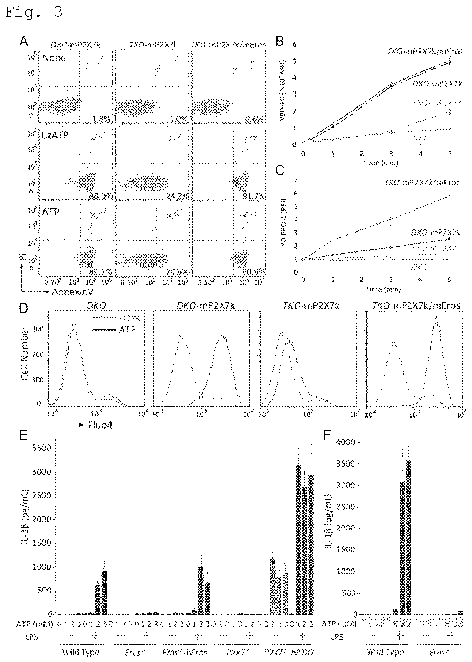

Fig. 3 shows an important role of Eros for P2X7-mediated signal transduction. (A) Effect of mEros on mP2X7k-mediated PtdSer exposure. (B and C) Effect of mEros on mP2X7k-mediated internalization of PtdCho and YO-PRO-1. (D) Effect of mEros on mP2X7k-mediated Ca²⁺ influx. (E) Effect of hEros on ATP-induced IL-1β secretion in THP-1 cells. (F) Requirement of Eros in ATP-induced IL-1β secretion in mouse bone marrow-derived macrophages (BMDMs).

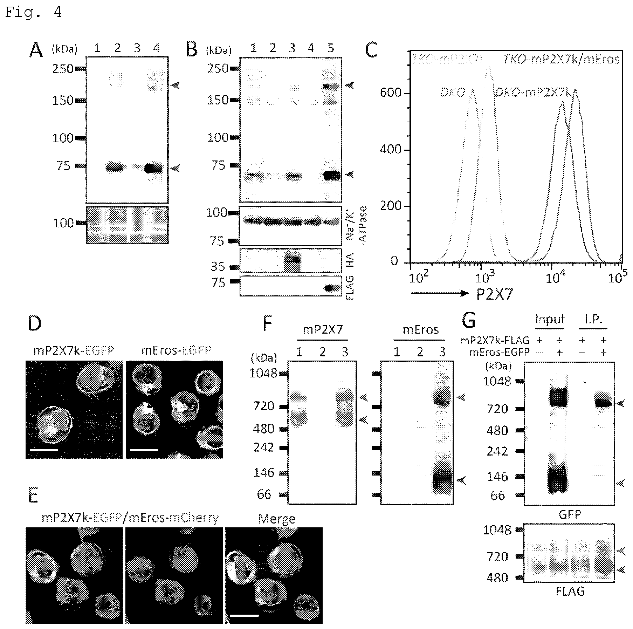

Fig. 4 shows the requirement of Eros for cell surface expression of P2X7. (A) Effect of mEros on the expression of exogenously introduced mP2X7k in WR19L cells. (B) Effect of hEros on endogenous P2X7 expression in THP-1 cells. (C) Effect of mEros on the cell surface expression of mP2X7k in WR19L cells. (D and E) Subcellular distribution of mP2X7k and mEros. (F) Results of BN-PAGE analysis of mP2X7 and mEros. (G) Results of coimmunoprecipitation of mEros and mP2X7k.

### Description of Embodiments

### 1. Definition

The terms "containing" and "comprising" as used herein include the concepts of "containing," "comprising," "consisting essentially of," and "consisting of."

The "identity" of amino acid sequences refers to the degree of consistency between two or more amino acid sequences that can be compared with each other. Thus, the higher the consistency between two amino acid sequences, the higher the identity or similarity between these sequences. Levels of amino acid sequence identity are determined using default parameters using, for example, FASTA, which is a sequence analysis tool. Alternatively, identity levels can be determined using the BLAST algorithm developed by Karlin and Altschul (Karlin S, Altschul SF, "Methods for assessing the statistical significance of molecular sequence features by using general scoring schemes," Proc Natl Acad Sci USA, 87: 2264-2268(1990); Karlin S, Altschul SF, "Applications and statistics for multiple high-scoring segments in molecular sequences," Proc Natl Acad Sci USA, 90: 5873-7 (1993)). A program called "BLASTX" based on the BLAST algorithm has been developed. Specific techniques for these analysis methods are known, and reference may be made to the National Center of Biotechnology Information (NCBI) website (http://www.ncbi.nlm.nih.gov/). The "identity" of base sequences is also defined as described above.

In the present specification, "conservative substitution" means that an amino acid residue is replaced by an amino acid residue having a similar side chain. Examples of conservative substitutions include substitution between amino acid residues having a basic side chain, such as lysine, arginine, and histidine. Other conservative substitutions include substitution between amino acid residues having an acidic side chain, such as aspartic acid and glutamic acid; substitution between amino acid residues having an uncharged polar side chain, such as glycine, asparagine, glutamine, serine, threonine, tyrosine, and cysteine; substitution between amino acid residues having a non-polar side chain, such as alanine, valine, leucine, isoleucine, proline, phenylalanine, methionine, and tryptophan; substitution between amino acid residues having a β-branched side chain, such as threonine, valine, and isoleucine; and substitution between amino acid residues having an aromatic side chain, such as tyrosine, phenylalanine, tryptophan, and histidine.

In the present specification, "nucleic acid" and "polynucleotide" are not particularly limited and encompass both natural and artificial ones. Specifically, in addition to DNA, RNA, and the like, those having a known chemical modification as described below may be used. To prevent degradation by hydrolases, such as nucleases, the phosphate residue (phosphate) of each nucleotide may be replaced with a chemically modified phosphate residue, such as phosphorothioate (PS), methylphosphonate, or phosphorodithionate. The hydroxyl group at position 2 of the sugar (ribose) of each ribonucleotide may be replaced with -OR (R representing, for example, CH₃ (2'-O-Me), CH₂CH₂OCH₃ (2'-O-MOE) , CH₂CH₂NHC (NH) NH₂, CH₂CONHCH₃, or CH₂CH₂CN) . Moreover, the base moiety (pyrimidine, purine) may be chemically modified; for example, a methyl group or a cationic functional group may be introduced at position 5 on a pyrimidine base, or the carbonyl group at position 2 may be changed to a thiocarbonyl group. Further, the phosphate moiety or hydroxyl moiety may be modified with, for example, biotin, an amino group, a lower alkyl amine group, or an acetyl group. However, chemical modification is not limited thereto. A BNA (LNA) and the like, in which the conformation of the sugar moiety is immobilized in the N form by bridging the 2'oxygen and 4'carbon in the sugar moiety of the nucleotide, can also be used.

### 2. P2X7 Receptor Expression Modulator

In one embodiment, the present invention relates to a P2X7 receptor expression modulator comprising at least one member selected from the group consisting of an Eros (essential for reactive oxygen species) expression modulator and a functional modulator of Eros (also referred to as "the modulator of the present invention" in the present specification). This is described below.

### 2-1. Modulation Target (Eros)

Eros protein and Eros mRNA, whose expression or function is to be modulated, are produced from the Eros (also referred to as CYBC1: cytochrome b-245 chaperone 1) gene, and are expressed in organisms or cells in which the expression of the P2X7 receptor is to be modulated. Thus, Eros protein and Eros mRNA to be suppressed vary depending on the target organism species. Examples of the organism species include, but are not limited to, animals, including various mammals, such as humans, monkeys, mice, rats, dogs, cats, rabbits, pigs, horses, cows, sheep, goats, and deer.

The amino acid sequences of Eros proteins derived from various organism species and the base sequences of Eros mRNAs derived from various organism species are known. Specifically, examples of human Eros protein include a protein comprising the amino acid sequence set forth in SEQ ID NO: 12 (NCBI Reference Sequence: NP_001093877.1), examples of mouse Eros protein include a protein comprising the amino acid sequence set forth in SEQ ID NO: 13 (NCBI Reference Sequence: NP_659081.1), examples of human Eros mRNA include an mRNA comprising the base sequence set forth in SEQ ID NO: 14 (NCBI Reference Sequence: NM_001100407.2), and examples of mouse Eros mRNA include an mRNA comprising the base sequence set forth in SEQ ID NO: 15 (NCBI Reference Sequence: NM_144832.2). Eros protein and Eros mRNA can also encompass splicing variants of the above.

Eros protein to be modulated may have amino acid mutations, such as substitution, deletion, addition, and insertion, as long as it has its original activity, i.e., molecular chaperone activity. For example, the mutation is preferably substitution, and more preferably conservative substitution, in terms of less susceptibility to loss of activity.

Eros mRNA to be modulated may have base mutations, such as substitution, deletion, addition, and insertion, as long as protein translated from the mRNA has its original activity, i.e., molecular chaperone activity. The mutation is preferably a mutation that does not cause amino acid substitution in protein translated from the mRNA or a mutation that causes amino acid conservative substitution in protein translated from the mRNA.

Preferable specific examples of Eros protein to be modulated include at least one member selected from the group consisting of a protein described in (a) below and a protein described in (b) below:
(a) a protein comprising the amino acid sequence set forth in SEQ ID NO: 12 or 13; and
(b) a protein comprising an amino acid sequence having 85% or more identity to the amino acid sequence set forth in SEQ ID NO: 12 or 13, and having molecular chaperone activity.

In (b) above, the identity is more preferably 90% or more, even more preferably 95% or more, and still even more preferably 98% or more.

Examples of the protein described in (b) above include the following:
(b') a protein comprising an amino acid sequence with substitution, deletion, addition, or insertion of one or more amino acids in the amino acid sequence set forth in SEQ ID NO: 12 or 13, and having molecular chaperone activity.

In (b') above, the number of "more amino acids" is, for example, 2 to 20, preferably 2 to 10, more preferably 2 to 5, and even more preferably 2 or 3.

Preferable specific examples of Eros mRNA to be modulated include at least one member selected from the group consisting of an mRNA described in (c) below and an mRNA described in (d) below:
(c) an mRNA comprising the base sequence set forth in SEQ ID NO: 14 or 15; and
(d) an mRNA comprising a base sequence having 85% or more identity to the base sequence set forth in SEQ ID NO: 14 or 15, and encoding a protein having molecular chaperone activity.

In (d) above, the identity is more preferably 90% or more, even more preferably 95% or more, and still even more preferably 98% or more.

Examples of the mRNA described in (d) above include the following:
(d') an mRNA comprising a base sequence with substitution, deletion, addition, or insertion of one or more bases in the base sequence set forth in SEQ ID NO: 14 or 15, and encoding a protein having molecular chaperone activity.

In (d') above, the number of "more bases" is, for example, 2 to 200, preferably 2 to 100, more preferably 2 to 50, and even more preferably 2 to 10.

### 2-2. Eros Expression Modulator

The Eros expression modulator is not particularly limited as long as it can modulate the expression of Eros protein or Eros mRNA expressed in an organism or cell in which the expression of the P2X7 receptor is to be modulated. The Eros expression modulator encompasses, for example, Eros expression suppressors and Eros expression promoters. The Eros expression modulators may be used singly or in a combination of two or more.

### 2-2-1. Eros Expression Suppressor

The Eros expression suppressor is not particularly limited as long as it can suppress the expression level of Eros protein, Eros mRNA, or the like. Examples include Eros-specific small interfering RNAs (siRNAs), Eros-specific microRNAs (miRNAs), Eros-specific antisense nucleic acids, and expression vectors thereof; Eros-specific ribozymes; Eros gene-editing agents used in a CRISPR/Cas system; and the like.

Expression suppression means that the expression levels of Eros protein, Eros mRNA, and the like are reduced to, for example, 1/2, 1/3, 1/5, 1/10, 1/20, 1/30, 1/50, 1/100, 1/200, 1/300, 1/500, 1/1000, or 1/10000 or less, and includes reducing these expression levels to 0.

### 2-2-1-1. siRNA, miRNA, Antisense Nucleic Acid, and Ribozyme

The Eros-specific siRNA is not particularly limited as long as it is a double-stranded RNA molecule that specifically suppresses the expression of a gene encoding Eros. In one embodiment, the siRNA preferably has a length of, for example, 18 bases or more, 19 bases or more, 20 bases or more, or 21 bases or more. The siRNA also preferably has a length of, for example, 25 bases or less, 24 bases or less, 23 bases or less, or 22 bases or less. It is envisaged that the upper and lower limits of the length of the siRNA mentioned here are combined arbitrarily. For example, the following combinations are envisaged: a length in which the lower limit is 18 bases, and the upper limit is 25 bases, 24 bases, 23 bases, or 22 bases; a length in which the lower limit is 19 bases, and the upper limit is 25 bases, 24 bases, 23 bases, or 22 bases; a length in which the lower limit is 20 bases, and the upper limit is 25 bases, 24 bases, 23 bases, or 22 bases; and a length in which the lower limit 21 bases, and the upper limit is 25 bases, 24 bases, 23 bases, or 22 bases.

The siRNA may be shRNA (small hairpin RNA). The shRNA can be designed so that part of it forms a stem loop structure. For example, assuming that the sequence of a certain region is designated as sequence a, and a strand complementary to sequence a is designated as sequence b, the shRNA can be designed to comprise sequence a, a spacer, and sequence b in this order on a single RNA strand and to have an overall length of 45 to 60 bases. Sequence a is the sequence of a partial region of the base sequence encoding the target Eros. The target region is not particularly limited, and any region can be used as a candidate. The length of sequence a is 19 to 25 bases, and preferably 19 to 21 bases.

The Eros-specific siRNA may have additional bases at the 5' or 3' end. The length of the additional bases is generally about 2 to 4 bases. The additional bases may be DNA or RNA. When DNA is used, the nucleic acid stability may improve. Examples of sequences of the additional bases include, but are not limited to, sequences such as ug-3', uu-3', tg-3', tt-3', ggg-3', guuu-3', gttt-3', ttttt-3', and uuuuu-3'.

The siRNA may have an overhang sequence at the 3' end. Specifically, for example, dTdT (wherein dT represents deoxythymidine) may be added. The siRNA may be blunt-ended without end addition. In the siRNA, the number of bases in the sense strand may be different from that in the antisense strand. For example, the siRNA may be asymmetrical interfering RNA (aiRNA), in which the antisense strand has overhang sequences at the 3' end and the 5' end. Typical aiRNA has an antisense strand composed of 21 bases and a sense strand composed of 15 bases, with a 3-base overhang at each end of the antisense strand.

The position of the target sequence for the Eros-specific siRNA is not particularly limited. In one embodiment, it is desirable to select a target sequence from a region other than the region between 5'-UTR and about 50 bases from the initiation codon, and other than the 3'-UTR region. It is preferred that target sequence candidates selected are examined for homology to a contiguous sequence of 16 to 17 bases in mRNA other than the target, using homology search software, such as BLAST (http://www.ncbi.nlm.nih.gov/BLAST/), to confirm the specificity of the target sequences selected. For a target sequence of which the specificity has been confirmed, double-stranded RNA composed of a sense strand having a 3'-end overhang of TT or UU in 19 to 21 bases after AA (or NA) and an antisense strand having a sequence complementary to the 19 to 21 bases and a 3'-end overhang of TT or UU may be designed as siRNA. Moreover, shRNA, which is a precursor of siRNA, can be designed by appropriately selecting any linker sequence (e.g., about 5 to 25 bases) that can form a loop structure and then connecting the above sense strand and antisense strand via the linker sequence.

The sequence of siRNA and/or shRNA can be searched using search software provided for free on various websites. Examples of such sites include the following:
siRNA Target Finder (http://www.ambion.com/jp/techlib/misc/siRNA_finder.html) provided by Ambion;
insert design tool for pSilencer (registered trademark);
Expression Vector (http://www.ambion.com/jp/techlib/misc/psilencer_converter.html) provided by Ambion; and
GeneSeer (http://codex.cshl.edu/scripts/newsearchhairpin.cgi) provided by RNAi Codex.

The siRNA can be prepared by synthesizing a sense strand and an antisense strand of a target sequence on mRNA using an automated DNA/RNA synthesizer and denaturing the strands in an appropriate annealing buffer at about 90 to 95°C for about 1 minute, followed by annealing at about 30 to 70°C for about 1 to 8 hours. The siRNA can also be prepared by synthesizing shRNA, which is a precursor of the siRNA, and cleaving the shRNA with RNA cleavage protein Dicer. As such Eros-specific siRNA, for example, SASI_Hs01_00242324, SASI_Hs02_00307980, or SASI_Mm01_00134930, all of which are sold by Merck, can be purchased and used.

The Eros-specific miRNA may be any miRNA as long as it inhibits translation of a gene encoding Eros. For example, the miRNA may inhibit translation of the gene by paring with 3' untranslated region (UTR) of the target rather than cleaving the target mRNA as in siRNA. The miRNA may be pri-miRNA (primary miRNA), pre-miRNA (precursor miRNA), or mature miRNA. The length of the miRNA is not particularly limited. The length of the pri-miRNA is generally several hundreds to several thousands of bases, the length of the pre-miRNA is generally 50 to 80 bases, and the length of the mature miRNA is generally 18 to 30 bases. In one embodiment, the Eros-specific miRNA is preferably pre-miRNA or mature miRNA, and more preferably mature miRNA. The Eros-specific miRNA may be synthesized by a known method or may be purchased from a company that provides synthetic RNA.

The Eros-specific antisense nucleic acid contains a base sequence complementary or substantially complementary to the base sequence of mRNA of a gene encoding Eros, or a part thereof, and has a function of suppressing synthesis of Eros protein by specifically binding to the mRNA to form a stable duplex. The antisense nucleic acid may be DNA, RNA, or DNA/RNA chimera. When the antisense nucleic acid is DNA, the RNA:DNA hybrid formed by the target RNA and the antisense DNA is recognized by endogenous ribonuclease H (RNase H) to cause selective degradation of the target RNA. Therefore, in the case of antisense DNA that directs degradation by RNase H, the target sequence may be not only a sequence in mRNA, but also the sequence of an intron region in the early transcription product of the Eros gene. The intron sequence can be determined by comparing the genome sequence with the cDNA base sequence of the Eros gene using a homology search program, such as BLAST or FASTA.

The length of the target region for the Eros-specific antisense nucleic acid is not limited as long as the translation into Eros protein is inhibited as a result of hybridization of the antisense nucleic acid. The target region for the Eros-specific antisense nucleic acid may be the whole sequence or a partial sequence of mRNA encoding Eros. Considering the ease of synthesis, antigenicity, transferability into cells, and other issues, an oligonucleotide of about 10 to 40 bases, particularly about 15 to 30 bases, is preferable, but this is not to be construed as limiting. More specifically, for example, the 5'-end hairpin loop, 5'-end untranslated region, translation initiation codon, protein coding region, ORF translation termination codon, 3'-end untranslated region, 3'-end palindrome region, or 3'-end hairpin loop of the Eros gene can be selected as a preferred target region for the antisense nucleic acid; however, the target region is not limited thereto.

The Eros-specific antisense nucleic acid may be one capable of not only hybridizing with the mRNA or early transcription product of the Eros gene to inhibit translation into protein, but also binding to the gene, which is double-stranded DNA, to form a triplex, thereby inhibiting transcription to RNA (antigene).

The Eros-specific siRNA, Eros-specific miRNA, Eros-specific antisense nucleic acid, and the like can be prepared by determining the target sequence of mRNA or early transcription product on the basis of the cDNA sequence or genomic DNA sequence of the Eros gene and synthesizing a sequence complementary thereto using a commercially available automated DNA/RNA synthesizer. All antisense nucleic acids containing various modifications can also be chemically synthesized by known methods.

The expression cassette of the Eros-specific siRNA, Eros-specific miRNA, or Eros-specific antisense nucleic acid is not particularly limited as long as it is a polynucleotide in which the Eros-specific siRNA, Eros-specific miRNA, or Eros-specific antisense nucleic acid is incorporated in an expressible state. Typically, the expression cassette comprises a polynucleotide containing a promoter sequence and a coding sequence for the Eros-specific siRNA, Eros-specific miRNA, or Eros-specific antisense nucleic acid (and further containing, optionally, a transcription termination signal sequence), and optionally comprises one or more other sequences. The promoter is not particularly limited, and examples include RNA polymerase II (pol II) promoters, such as a CMV promoter, an EF-1α promoter, an SV40 promoter, an MSCV promoter, an hTERT promoter, a β-actin promoter, and a CAG promoter; RNA polymerase III (pol III) promoters, such as mouse and human U6-snRNA promoters, a human H1-RNase P RNA promoter, and a human valine-tRNA promoter; and the like. Among these, pol III promoters are preferable in terms of achieving accurate transcription of short RNA. Various promoters that are inducible by agents can also be used. The other sequences are not particularly limited, and various known sequences that can be contained in expression vectors can be used. Examples of such sequences include origin of replication, a drug resistance gene, and the like. Examples of the type of drug resistance gene and the type of vector include those described above.

Another example of the Eros expression suppressor is an Eros-specific ribozyme or the like. Although "ribozyme" refers to, in a narrow sense, RNA having enzyme activity to cleave nucleic acids, the term "ribozyme" as used herein also encompasses DNA as long as it has sequence-specific nucleic acid cleavage activity. The most versatile ribozyme nucleic acid is self-splicing RNA found in infectious RNA such as a viroid or a virusoid, and the hammerhead type, hairpin type, and the like are known. The hammerhead type exhibits enzyme activity with about 40 bases in length, and can specifically cleave only the target mRNA by making several bases at both ends adjacent to the hammerhead structure portion (about 10 bases in total) complementary to the desired cleavage site of the mRNA. This type of ribozyme nucleic acid uses only RNA as a substrate, and thus has an advantage in that it does not attack genomic DNA. When the mRNA of the Eros gene has a double-stranded structure by itself, the target sequence can be made to be single-stranded by using a hybrid ribozyme to which an RNA motif derived from a viral nucleic acid and capable of binding specifically to an RNA helicase is linked (Proc. Natl. Acad. Sci. USA, 98(10): 5572-5577 (2001)). Furthermore, when a ribozyme is used in the form of an expression vector containing DNA encoding the ribozyme, the ribozyme may be a hybrid ribozyme to which a sequence obtained by modifying tRNA is further linked to promote the transfer of the transcription product to cytoplasm (Nucleic Acids Res., 29(13): 2780-2788 (2001)).

### 2-2-1-2. Gene-Editing Agent

The Eros gene-editing agent is not particularly limited as long as the expression of the Eros gene can be suppressed by a target sequence-specific nuclease system (e.g., CRISPR/Cas system). The expression of the Eros gene can be suppressed, for example, by disrupting the Eros gene or suppressing the activity of a promoter of the Eros gene by modifying the promoter.

When, for example, a CRISPR/Cas system is used, a vector (vector for Eros gene editing) containing a guide RNA expression cassette that targets the Eros gene or a promoter thereof and a Cas protein expression cassette can be typically used as the Eros gene-editing agent; however, the Eros gene-editing agent is not limited thereto. In addition to this typical example, for example, a combination of a vector containing a guide RNA targeting the Eros gene or a promoter thereof and/or an expression cassette thereof and a vector containing a Cas protein expression cassette and/or an expression cassette thereof can be used as the Eros gene-editing agent.

The guide RNA expression cassette is not particularly limited as long as it is a polynucleotide used for the purpose of expressing guide RNA in an organism to be metabolically improved. Typical examples of the expression cassette include a polynucleotide containing a promoter and a coding sequence for some or all of guide RNA placed under the control of the promoter. The phrase "placed under the control of the promoter" means, in other words, that the coding sequence for guide RNA is placed so that the transcription of the sequence is controlled by the promoter. In a specific embodiment, for example, the coding sequence for guide RNA is placed immediately downstream of the 3'-side of the promoter (e.g., the number of base pairs (bp) between the base at the 3' end of the promoter and the base at the 5' end of the coding sequence for guide RNA is, for example, 100 bp or less, and preferably 50 bp or less).

The promoter of the guide RNA expression cassette is not particularly limited. Pol II promoters can be used, but pol III promoters are preferable in terms of achieving more accurate transcription of relatively short RNA. Examples of usable pol III promoters include, but are not limited to, mouse and human U6-snRNA promoters, a human H1-RNase P RNA promoter, a human valine-tRNA promoter, and the like. Various promoters that are inducible by agents can also be used.

The coding sequence for guide RNA is not particularly limited as long as it is a base sequence encoding guide RNA.

The guide RNA is not particularly limited as long as it is used in a CRISPR/Cas system. For example, various guide RNAs capable of binding to the target site of genomic DNA (e.g., Eros gene or a promoter thereof) and binding to Cas protein to guide the Cas protein to the target site of the genomic DNA can be used.

The phrase "target site" as used herein is a site on genomic DNA that is composed of a DNA strand (target strand) composed of a PAM (protospacer adjacent motif) sequence and a sequence of about 17 to 30 bases in length (preferably 18 to 25 bases in length, more preferably 19 to 22 bases in length, and particularly preferably 20 bases in length) adjacent to its 5'-side; and a complementary DNA strand (non-target strand) thereof.

The PAM sequence varies depending on the type of Cas protein used. For example, the PAM sequence corresponding to the Cas9 protein derived from *S. pyogenes* (type II) is 5'-NGG, the PAM sequence corresponding to the Cas9 protein derived from *S*. *solfataricus* (type I-A1) is 5'-CCN, the PAM sequence corresponding to the Cas9 protein derived from *S. solfataricus* (type I-A2) is 5'-TCN, the PAM sequence corresponding to the Cas9 protein derived from *H. walsbyl* (type I-B) is 5'-TTC, the PAM sequence corresponding to the Cas9 protein derived from *E. coli* (type I-E) is 5'-AWG, the PAM sequence corresponding to the Cas9 protein derived from *E. coli* (type I-F) is 5'-CC, the PAM sequence corresponding to the Cas9 protein derived from P. *aeruginosa* (type I-F) is 5'-CC, the PAM sequence corresponding to the Cas9 protein derived from *S. thermophilus* (type II-A) is 5'-NNAGAA, the PAM sequence corresponding to the Cas9 protein derived from *S. agalactiae* (type II-A) is 5'-NGG, the PAM sequence corresponding to the Cas9 protein derived from *S. aureus* is 5'-NGRRT or 5'-NGRRN, the PAM sequence corresponding to the Cas9 protein derived from *N*. *meningitidis* is 5'-NNNNGATT, and the PAM sequence corresponding to the Cas9 protein derived from T. *denticola* is 5'-NAAAAC.

The guide RNA has a sequence involved in binding to the target site of genomic DNA (sometimes referred to as a "crRNA (CRISPR RNA) sequence"). When the crRNA sequence binds complementarily (preferably, in a complementary and specific manner) to the sequence of the non-target strand excluding the sequence complementary to the PAM sequence, the guide RNA can bind to the target site of genomic DNA.

Binding "complementarily" includes not only the case of binding based on perfect complementarity (A and T, and G and C), but also the case of binding based on complementarity to a degree that allows hybridization under stringent conditions. The stringent conditions can be determined based on the melting temperature (Tm) of the nucleic acid binding a complex or probe, as taught in Berger and Kimmel (1987, Guide to Molecular Cloning Techniques Methods in Enzymology, Vol. 152, Academic Press, San Diego CA). For example, the washing conditions after hybridization generally include about "1×SSC, 0.1% SDS, 37°C." It is preferable that the hybridization state is maintained even when washing is performed under such conditions. Examples of the washing conditions also include, but are not limited to, stricter hybridization conditions of about "0.5×SSC, 0.1%SDS, 42°C" and even stricter hybridization conditions of about "0.1×SSC, 0.1%SDS, 65°C."

Specifically, in the crRNA sequence, a sequence that binds to the target sequence has, for example, 90% or more, preferably 95% or more, more preferably 98% or more, even more preferably 99% or more, and particularly preferably 100% identity to the target strand. It is said that in the crRNA sequence, 12 bases on the 3'-side of the sequence that binds to the target sequence are important for the binding of the guide RNA to the target site. Thus, in the crRNA sequence, when the sequence that binds to the target sequence is not completely identical to the target strand, the base(s) different from those of the target strand is/are preferably base(s) other than the 12 bases on the 3'-side of the sequence that binds to the target sequence, in the crRNA sequence.

The guide RNA has a sequence involved in binding to the Cas protein (sometimes referred to as a "tracrRNA (transactivating crRNA) sequence"). When the tracrRNA sequence binds to the Cas protein, the guide RNA can guide the Cas protein to the target site of genomic DNA.

The tracrRNA sequence is not particularly limited. The tracrRNA sequence is typically an RNA composed of a sequence of about 50 to 100 bases in length capable of forming multiple (generally three) stem loops, and the sequence varies depending on the type of Cas protein used. As the tracrRNA sequence, various known sequences can be used depending on the type of Cas protein used.

The guide RNA generally contains the crRNA sequence and the tracrRNA sequence. The embodiment of the guide RNA may be a single-stranded RNA (sgRNA) containing the crRNA sequence and the tracrRNA sequence, or may be an RNA complex formed by complementarily binding an RNA containing the crRNA sequence and an RNA containing the tracrRNA sequence.

The Cas protein expression cassette is not particularly limited as long as it is a polynucleotide used for the purpose of expressing the Cas protein in the target organisms in which the metabolism is to be improved. Typical examples of the expression cassette include a polynucleotide containing a promoter and a coding sequence for the Cas protein placed under the control of the promoter. The phrase "placed under the control of the promoter" is as defined in the explanation of the guide RNA expression cassette.

The promoter of the Cas protein expression cassette is not particularly limited, and for example, various pol II promoters can be used. Examples of pol II promoters include, but are not limited to, a CMV promoter, an EF-1α promoter, an SV40 promoter, an MSCV promoter, an hTERT promoter, a β-actin promoter, a CAG promoter, and the like. Various promoters that are inducible by agents can also be used.

The coding sequence for the Cas protein is not particularly limited as long as it is a base sequence encoding the amino acid sequence of the Cas protein.

The Cas protein is not particularly limited as long as it is used in a CRISPR/Cas system. For example, various Cas proteins capable of binding to a target site of genomic DNA while forming a complex with guide RNA, and cleaving the target site can be used. Cas proteins derived from various organisms are known, and examples include the Cas9 protein derived from *S*. *pyogenes* (type II), the Cas9 protein derived from *S. solfataricus* (type I-A1), the Cas9 protein derived from *S. solfataricus* (type I-A2), the Cas9 protein derived from *H. walsbyl* (type I-B), the Cas9 protein derived from *E. coli* (type I-E), the Cas9 protein derived from *E. coli* (type I-F), the Cas9 protein derived from P. *aeruginosa* (type I-F), the Cas9 protein derived from *S*. *thermophilus* (type II-A), the Cas9 protein derived from *S*. *agalactiae* (type II-A), the Cas9 protein derived from *S. aureus,* the Cas9 protein derived from *N*. *meningitidis,* the Cas9 protein derived from *T. denticola,* the Cpf1 protein derived from F. *novicida* (type V), and the like. Of these, Cas9 proteins are preferable, and, for example, Cas9 proteins endogenously present in bacteria belonging to the genus *Streptococcus* are more preferable. Information on the amino acid sequences of various Cas proteins and their coding sequences can be easily obtained from various databases such as NCBI.

The Cas protein may be a wild-type double-strand break-generating Cas protein or a nickase-type Cas protein. The Cas protein may have mutations (e.g., substitution, deletion, insertion, or addition) in the amino acid sequence as long as its activity is not impaired. A known protein tag, a signal sequence, or a protein such as an enzyme protein may be added to the Cas protein. Examples of protein tags include biotin, a His tag, a FLAG tag, a Halo tag, an MBP tag, an HA tag, a Myc tag, a V5 tag, a PA tag, and the like. Examples of signal sequences include cytoplasm translocation signals and the like.

The Eros gene-editing vector may have other sequences. The other sequences are not particularly limited, and various known sequences that can be contained in expression vectors can be used. Examples of such sequences include origin of replication, a drug resistance gene, and the like.

Examples of drug resistance genes include chloramphenicol resistance genes, tetracycline resistance genes, neomycin resistance genes, erythromycin resistance genes, spectinomycin resistance genes, kanamycin resistance genes, hygromycin resistance genes, puromycin resistance genes, and the like.

The type of vector is not particularly limited, and examples include plasmid vectors such as animal cell expression plasmids; virus vectors such as retroviruses, lentiviruses, adenoviruses, adeno-associated viruses, herpes viruses, and Sendai viruses; *Agrobacterium* vectors; and the like.

The Eros gene-editing agent can be easily prepared according to a known genetic engineering method. For example, the Eros gene-editing agent can be prepared using PCR, restriction enzyme cleavage, a DNA ligation technique, an in vitro transcription/translation technique, a recombinant protein production technique, etc.

### 2-2-2. Eros Expression Promoter

The Eros expression promoter is not particularly limited as long as it can increase the amount of Eros in cells.

Examples of the Eros expression promoter include an Eros expression cassette. The Eros expression cassette is not particularly limited as long as Eros is incorporated in an expressible state. Typically, the Eros expression cassette comprises a polynucleotide containing a promoter sequence and a coding sequence for Eros (and further containing, optionally, a transcription termination signal sequence). The expression cassette may also be in the form of a vector.

The expression vector is not particularly limited, and examples include plasmid vectors such as animal cell expression plasmids; virus vectors such as retroviruses, lentiviruses, adenoviruses, adeno-associated viruses, herpes viruses, and Sendai viruses; and the like.

The promoter is not particularly limited, and examples include a CMV promoter, an EF-1α promoter, an SV40 promoter, an MSCV promoter, an hTERT promoter, a β-actin promoter, a CAG promoter, and the like. Various promoters that are inducible by agents can also be used.

The expression vector may also contain other elements that can be contained in expression vectors, in addition to the above. Examples of the other elements include origin of replication, a drug resistance gene, and the like. Examples of drug resistance genes include, but are not limited to, chloramphenicol resistance genes, tetracycline resistance genes, neomycin resistance genes, erythromycin resistance genes, spectinomycin resistance genes, kanamycin resistance genes, hygromycin resistance genes, puromycin resistance genes, and the like.

The Eros expression vector can be easily prepared according to a known genetic engineering method. For example, the Eros expression vector can be prepared using PCR, restriction enzyme cleavage, a DNA ligation technique, etc.

Other examples of the Eros expression promoter include a transcriptional activator for Eros and its expression vector, a low molecular weight compound capable of activating the transcription of Eros, and the like. The embodiment of the expression vector is as described in the explanation of the Eros expression vector.

### 2-3. Functional Modulator of Eros

The functional modulator of Eros is not particularly limited as long as it can modulate the function of Eros protein or Eros mRNA expressed in an organism or cell in which the expression of the P2X7 receptor is to be modulated. The functional modulator of Eros encompasses, for example, Eros function suppressors, Eros function promoters, and the like. The functional modulators of Eros may be used singly or in a combination of two or more.

The functional modulator of Eros is not particularly limited as long as it can reduce molecular chaperone activity, especially molecular chaperone activity for the P2X7 receptor.

Examples of the functional modulator of Eros include Eros neutralizing antibodies and the like. The Eros neutralizing antibody refers to an antibody that has a property of inhibiting the molecular chaperone activity of Eros by binding to Eros.

The antibody encompasses polyclonal antibodies, monoclonal antibodies, chimeric antibodies, single-stranded antibodies, and a part of the antibodies having an antigen-binding property, such as Fab fragments and fragments produced by a Fab expression library. The antibody of the present invention also encompasses antibodies that show antigen binding to a polypeptide of generally at least 8 contiguous amino acids, preferably at least 15 contiguous amino acids, more preferably at least 20 contiguous amino acids of the Eros amino acid sequence.

In order to more reliably exhibit neutralization activity, the antibody is preferably an antibody that shows antigen binding to the amino acid sequence of the binding site of Eros to the P2X7 receptor. The binding site can be determined based on known information and/or inferred based on known information (e.g., by building a docking model).

Production methods for these antibodies are known, and the antibody of the present invention can be produced using such usual methods (Current Protocols in Molecular Biology, Chapter 11.12 to 11.13 (2000)). Specifically, when the antibody of the present invention is a polyclonal antibody, the antibody can be obtained according to a usual method from the serum of an immunized animal produced by immunizing a non-human animal, such as a domesticated rabbit, with Eros purified after being expressed in, for example, *Escherichia coli* using a usual method, or with an oligopeptide synthesized using a usual method and including a partial amino acid sequence of the Eros. In the case of a monoclonal antibody, the antibody can be obtained from hybridoma cells prepared by fusing spleen cells and myeloma cells obtained by immunizing a non-human animal, such as a mouse, with Eros purified after being expressed in, for example, *Escherichia coli* using a usual method, or with an oligopeptide including a partial amino acid sequence of Eros (Current Protocols in Molecular Biology, edited By Ausubel et al. (1987) Published by John Wiley and Sons. Section 11.4 to 11.11). Moreover, multiple antibodies are available. For example, Anti-Eros antibody ab150936 sold by Abcam, Anti-C17orf62 antibody HPA045696 sold by Atlas Antibodies, and the like are known.

Eros used as an immunogen for the production of the antibody can be obtained according to procedures such as DNA cloning, construction of plasmids, transfection into a host, culturing a transformant, and collection of the protein from the culture, based on known gene sequence information. These procedures can be performed according to methods known to those skilled in the art or methods described in documents (e.g., Molecular Cloning, T. Maniatis et al., CSH Laboratory (1983), DNA Cloning, DM. Glover, IRL PRESS (1985)).

Specifically, the protein as an immunogen for the production of the antibody of the present invention can be obtained by producing recombinant DNA (expression vector) that allows a gene encoding Eros to be expressed in a desired host cell, introducing the recombinant DNA into a host cell to obtain a transformant, culturing the transformant, and collecting the target protein from the resulting culture. A partial peptide of Eros can be produced by a common chemical synthesis (peptide synthesis) method according to known gene sequence information.

The antibody of the present invention may be prepared by using an oligopeptide having a partial amino acid sequence of Eros. The oligo(poly)peptide used to produce the antibody does not need to have functional biological activity, but desirably has immunogenic characteristics similar to those of Eros. A preferred example is an oligo(poly)peptide that has such immunogenic characteristics and that comprises at least 8 contiguous amino acids, preferably 15 contiguous amino acids, more preferably 20 contiguous amino acids of the Eros amino acid sequence.

The antibody against the oligo(poly)peptide also may be produced by enhancing an immunological reaction with various adjuvants selected according to the type of host. Examples of adjuvants include, but are not limited to, Freund's adjuvant, mineral gel such as aluminum hydroxide, surface active materials such as lysolecithin, pluronic polyol, polyanion, peptide, oil emulsion, keyhole limpet hemocyanin, and dinitrophenol, and human adjuvants such as BCG (bacillus Calmette-Guérin) and *Corynebacterium parvum.*

In addition to the above Eros neutralizing antibodies, Eros antagonists, Eros agonists, Eros dominant negative mutants, and the like can be used as the functional modulator of Eros. When a protein such as a neutralizing antibody is used as the functional modulator of Eros, an expression cassette thereof can also be used instead of the protein. The expression cassette is as defined in the "2-2. Eros Expression Modulator" section above.

### 2-4. Use and Other Components

As will be clarified in the Examples described later, Eros, a membrane protein localized in the endoplasmic reticulum membrane, is a chaperone molecule essential for stable expression of the P2X7 homotrimer and functions by physically interacting with P2X7 synthesized in the endoplasmic reticulum. In cells in which the Eros-P2X7 interaction is impaired, it is difficult for P2X7 to fold properly, resulting in a marked reduction in the expression level of P2X7. On the other hand, in cells expressing Eros in a large amount, the proper folding of P2X7 is promoted, resulting in increases in the total amount of P2X7 and the expression level of P2X7 on the plasma membrane.

Thus, at least one member (active ingredient) selected from the group consisting of an Eros expression modulator and a functional modulator of Eros has a P2X7 receptor expression modulating action and can therefore be used as an active ingredient in P2X7 receptor expression modulators (e.g., medicaments, reagents, food compositions, oral compositions, health enhancers, and nutrition supplements (e.g., supplements)). The active ingredient can be applied to animals, humans, and various cells (for example, by administration, ingestion, or inoculation) directly or as various compositions formed together with common components.

The modulator of the present invention can be used for modulating various phenomena associated with P2X7 receptor modulation. For example, the modulator of the present invention can suppress P2X7 receptor expression by suppressing Eros expression and/or function, thereby suppressing production of IL-1β. For example, the modulator of the present invention can suppress P2X7 receptor expression by suppressing Eros expression and/or function, thereby preventing or ameliorating inflammation (in one embodiment, nitric oxide-independent inflammation, although there is no particular limitation), pain, and the like, more specifically, for example, articular rheumatism, osteoarthritis, interstitial cystitis, interstitial fibrosis, psoriasis, septic shock, sepsis, allergic dermatitis, asthma, allergic asthma, mild to severe asthma, steroid-resistant asthma, idiopathic pulmonary fibrosis, allergic rhinitis, chronic obstructive pulmonary disease, airway hyperresponsiveness, acute and chronic pain, neuropathic pain, inflammatory pain, migraine, spontaneous pain, opioid-induced pain, diabetic neuropathy, postherpetic neuralgia, lower-back pain, chemotherapy-induced neuropathic pain, fibromyalgia, neuropathic pain, mood disorder, major depression, major depressive disorder, treatment-resistant depression, bipolar disorder, anxious depression, anxiety, cognition, sleep disorder, multiple sclerosis, epileptic seizure, Parkinson's disease, schizophrenia, Alzheimer's disease, Huntington's disease, amyotrophic lateral sclerosis, autism, spinal cord injury, cerebral ischemia/traumatic brain injury, stress-related disorder, diabetes, diabetes mellitus, thrombosis, inflammatory bowel disease, irritable bowel disease, irritable bowel syndrome, Crohn's disease, cardiovascular disease (examples of cardiovascular disease include hypertension, myocardial infarction, ischemic heart disease, ischemia, and the like), ureteral obstruction, lower urinary tract syndrome, lower urinary tract dysfunction (e.g., incontinence), disease after cardiac transplantation, osteoporosis/osteopetrosis, diseases involved in the secretory function of exocrine glands, glaucoma, nephritis, glomerulonephritis, Chagas disease, chlamydia, neuroblastoma, tuberculosis, polycystic kidney disease, cancer, acne, and the like. As another example, the modulator of the present invention can promote P2X7 receptor expression by promoting Eros expression and/or function, thereby promoting differentiation and/or maintenance of memory T cells.

The target cells for the modulator of the present invention are not particularly limited as long as they express the P2X7 receptor (exogenous or endogenous). Examples include immune cells (e.g., T cells), vascular endothelial cells, endothelial progenitor cells, stem cells (e.g., bone marrow-derived stem cells, adipose tissue-derived stem cells, mesenchymal stem cells, pluripotent stem cells (iPS cells, ES cells, and the like)), muscle cells (skeletal muscle cells, smooth muscle cells, and cardiomyocytes), muscle progenitor cells (e.g., cardiac progenitor cells and myoblasts), nerve cells, and the like.

The target organism for the modulator of the present invention is not particularly limited, and examples include various mammals, such as humans, monkeys, mice, rats, dogs, cats, rabbits, pigs, horses, cows, sheep, goats, and deer.

The form of the modulator of the present invention is not particularly limited, and can take a form generally used in each use depending on the intended use of the modulator of the present invention.

Examples of the form of the modulator of the present invention for use as medicaments, health enhancer, nutrition supplements (e.g., supplements), and the like include dosage forms suitable for oral administration (oral preparations), such as tablets (including orally disintegrating tablets, chewable tablets, effervescent tablets, troches, jelly drops, etc.), pills, granules, fine granules, powders, hard capsules, soft capsules, dry syrups, liquids (including drinkable preparations, suspensions, and syrups), and jellies; and dosage forms suitable for parenteral administration (parenteral preparations), such as nasal drops, inhalants, rectal suppositories, inserts, enemas, jellies, injections, patches, lotions, and creams.

Examples of the form of the modulator of the present invention for use as food compositions include liquid, gel, or solid foods, such as juices, soft drinks, teas, soups, soybean milk, salad oils, dressings, yoghurts, jellies, puddings, *furikake* (dry Japanese seasoning), powdered infant milk, cake mixes, powdered or liquid dairy products, bread, cookies, and the like.

Examples of the form of the modulator of the present invention for use as oral compositions include liquids (e.g., solutions, emulsions, and suspensions), semi-solids (e.g., gels, creams, and pastes), solids (e.g., tablets, granules, capsules, film agents, kneaded materials, molten solids, wax-like solids, and elastic solids), and any like forms. Specific examples include dentifrices (e.g., toothpastes, dental rinses, liquid dentifrices, and tooth powder), mouth washes, ointments, patches, mouth deodorants, foods (e.g., chewing gum, tablets, candies, gummy candies, films, and troches), and the like.

The modulator of the present invention may further contain other components, if necessary. The other components are not particularly limited as long as they can be incorporated into medicaments, food compositions, oral compositions, health enhancers, nutrition supplements (e.g., supplements), etc. Examples include bases, carriers, solvents, dispersants, emulsifiers, buffers, stabilizers, excipients, binders, disintegrators, lubricants, thickeners, moisturizers, coloring agents, flavoring agents, chelating agents, and the like.

The content of the active ingredient in the modulator of the present invention varies depending on, for example, the type of active ingredient, the use, the mode of use, the target of application, and the condition of the target, and is not limited. The content of the active ingredient is, for example, 0.0001 to 100 wt.%, and preferably 0.001 to 50 wt.%.

The amount of application (e.g., administration, ingestion, or inoculation) of the modulator of the present invention is not particularly limited as long as it is a pharmaceutically effective amount. In terms of the weight of the active ingredient, the amount is generally 0.1 to 1000 mg/kg of body weight per day. This dose is preferably administered once a day, or in two or three portions a day, and can be suitably increased or decreased according to the age, disease state, and symptoms.

### 3. Cell

In one embodiment, the present invention relates to a cell in which the modulator of the present invention has been introduced (also referred to as "the cell of the present invention" in the present specification).

As described above, in one embodiment, the modulator of the present invention can promote differentiation and/or maintenance of memory T cells by promoting Eros expression and/or function. Thus, when the cell of the present invention containing at least one member selected from the group consisting of an Eros expression promoter and an Eros function promoter is a T cell (the T cell of the present invention), the cell of the present invention can be suitably used as a T cell for use in CAR-T therapies.

In order to prevent CAR-T cells from remaining in the patient's body for a long period of time after therapy, the T cell of the present invention is preferably a cell that can modulate Eros expression and/or Eros function so that the Eros expression and/or Eros function is transiently enhanced. As a method for such modulation, for example, an expression cassette in which the expression is modulated by a drug-responsive promoter can be used as an Eros expression and/or Eros function promoter to transiently enhance Eros expression and/or Eros function by addition of the drug.

Moreover, from the same viewpoint, it is preferable that in the T cell of the present invention, the modulater of the present invention containing at least one member selected from the group consisting of an Eros expression suppressor and an Eros function suppressor has been further introduced. This allows Eros expression and/or Eros function to be modulated so that the Eros expression and/or Eros function is suppressed after transiently enhancing the Eros expression and/or Eros function, for example by using a drug-responsive promoter.

### Examples

The present invention is described below in detail with reference to Examples; however, the present invention is not limited to these Examples.

### Materials and Methods

The materials and methods of the experiments performed in the following Test Examples are as shown below unless otherwise specified.

### Material and Method 1: Cell Line, Plasmid, Antibody, and Reagent

Mouse WR19L cells (ATCC TIB-52) were grown in RPMI 1640 supplemented with 10% FCS. Human THP-1 cells (ATCC TIB-202) were cultured in RPMI 1640 supplemented with 10% FCS and 55 µM β-mercaptoethanol. HEK293T cells (ATCC CRL-1573) were grown in DMEM containing 10% FCS.

A pNEF-BOS vector was derived from pEF-BOS (Nucleic Acids Res. 18, 1990: 5322) into which an SV40 early promoter-driven neomycin resistance gene was introduced from pSV2-neo (Nature 302, 1983: 340-342). A LentiCas9-Blast vector (Nat. Meth. 11, 2014: 783-784), Mouse CRISPR Knockout Pooled Library (GeCKO v2) (Nat. Meth. 11, 2014: 783-784), and pX330 and pX459v2 plasmids (Nat. Protoc. 8, 2013: 2281-2308) were obtained from Addgene. pCMV-VSV-G-RSV-rev and pCMV-VSV-G (J. Virol. 72, 1998: 8150-8157) were provided by Riken Bioresource Center. A pMXspuro retroviral vector (Exp. Hematol. 31, 2003: 1007-1014) and pGag-pol-IRES-bsr packaging plasmid (Gene Ther. 7, 2000: 1063-1066) were obtained from the Institute of Medical Science, the University of Tokyo. pAdVAntage and pLVSIN-EF-1α lentiviral vectors were purchased from Thermo Fisher Scientific and Takara Bio, respectively.

Alexa 488-conjugated rat anti-mouse P2X7 mAb (clone Hano 43) was obtained from Bio-Rad. HRP mouse anti-FLAG M2 mAb, anti-FLAG M2-conjugated magnetic beads, and 3×FLAG peptide were obtained from Merck. HRP-rabbit anti-GFP Ab was obtained from Medical & Biological Laboratories. HRP mouse anti-HA mAb (clone 16B12) was obtained from BioLegend. Rabbit anti-P2X7 Ab was obtained from Alomone Labs. HRP goat anti-rabbit IgAb was obtained from Agilent Technologies. 2'(3')-O-(4-Benzoylbenzoyl)adenosine 5'-triphosphate (BzATP) and ATP were purchased from Wako Pure Chemical and Nacalai Tesque, respectively. O,O'-Bis(2-aminophenyl)ethyleneglycol-N,N,N',N'-tetraacetic acid, tetraacetoxymethyl ester (BAPTA-AM), and 1-[2-amino-5-(2,7-difluoro)-6-acetoxymethoxy-3-oxo-9-xanthenyl)phenoxy]-2- (2-amino-5-methylphenoxy)ethane-N,N,N',N' - tetraacetic acid, tetra(acetoxymethyl)ester (Fluo 4-AM) were obtained from Dojindo. Cy5-labeled annexin V was obtained from BioVision. Ionomycin and propidium iodide (PI) were obtained from Merck. SYTOX Blue, YO-PRO-1, and Hoechst 33342 were obtained from Thermo Fisher Scientific. 1-Oleoyl-2-f6-[(7-nitro-2-1,3-benzoxadiazol-4-yl)amino]hexanoyl}-glycero-3-phosphocholine (NBD-PC) was obtained from Avanti Polar Lipids.

### Material and Method 2: Genome Editing and Transformation of Cell Line

TMEM16F, P2X7, and Eros genes were knocked out using a CRISPR-Cas9 system and pX330 or pX459v2 plasmid, as previously reported (Science 344, 2014: 1164-1168, Nat. Protoc. 8, 2013: 2281-2308). Complementary oligonucleotides carrying the sgRNA target sequences were as follows: mouse (m)TMEM16F, GGATGAAGTCGATTCGCCTC (SEQ ID NO: 1); mP2X7, TGAGCGATAAGCTGTACCAG (SEQ ID NO: 2); mEros, ATTTGTGGCTGTACAGAACT (SEQ ID NO: 3); human (h)P2X7, TGATGACAGGCTCTTTCCGC (SEQ ID NO: 4); and hEros, TGGAAGCTCTTCTACGTCAC (SEQ ID NO: 5). The oligonucleotides were ligated into pX330 or pX459v2, and the resulting plasmid DNA was introduced into WR19L or THP-1 cells by electroporation using an NEPA21 Super Electroporator (NepaGene). In some cases, transfection was performed twice at a 3-day interval. 20 to 30 hours after transfection with the pX459v2 vector, the cells were treated with 1 µg/mL puromycin for 30 hours. Single clones were isolated by limiting dilution and genotyped by sequencing the sgRNA target regions of the corresponding chromosomal genes.

To express P2X7 and Eros, the coding sequences for mP2X7k (FJ436444), mEros (NM_144832), hP2X7a (NM_002562), and hEros (NM_001100407) were prepared by RT-PCR using RNA from WR19L (mP2X7k and mEros) or THP-1 (hP2X7a and hEros). These cDNAs were tagged at the C-terminus with FLAG, HA, EGFP, or mCherry and inserted into pNEF-BOS (mP2X7k), pMXs-puro (mP2X7k and mEros), or pLVSIN-EF-1α (hP2X7a and hEros). The authenticity of the expression plasmids was confirmed by DNA sequencing. The expression plasmid constructed using the pNEF-BOS vector was digested with one restriction enzyme site on the vector and introduced into WR19L cells by electroporation. Stable transformants were selected by culturing in the presence of 2 mg/mL G418. For viral transformation, the pMXs-puro-based vectors were introduced into HEK293T cells together with pGag-pol-IRES-bsr, pCMV-VSV-G, and pAdVAntage, and the pLVSIN-EF-1α-based vectors were introduced together with pCAG-HIVgp and pCAG-HIVgp. Retroviruses and lentiviruses in the pCMV-VSV-G-RSV-rev culture supernatant were concentrated at 4°C by centrifugation at 6000×g for 16 hours and used to infect WR19L and THP-1 cells, respectively. Transformants were selected in the presence of 1 µg/mL puromycin. If necessary, mP2X7-, EGFP-, or mCherry-positive cells were sorted using FACSAria^{™}II (BD Biosciences).

### Material and Method 3: Flow Cytometry for mP2X7, PtdSer Exposure, NBD-PC, YO-PRO-1, and Intracellular Ca²⁺

To detect mP2X7 on WR19L cell transformants, 5×10⁵ cells were washed with PBS containing 2% FCS (PBS/FCS) and incubated in 100 µL of PBS/FCS containing a 40-fold diluted Alexa488-anti-mP2X7 antibody on ice for 30 minutes. The cells were then washed with PBS/FCS, suspended in 250 µL of PBS/FCS containing 250 nM SYTOX Blue, and analyzed by flow cytometry using FACSCanto^{™} II (BD Biosciences). The data were analyzed with FlowJo software (BD Biosciences).

P2X7-mediated PtdSer exposure was analyzed by annexin V binding, followed by flow cytometry. Specifically, 1.8×10⁶ cells were washed with PBS, resuspended in annexin buffer (10 mM HEPES-NaOH (pH 7.5), 140 mM NaCl, and 2.5 mM CaCl₂) containing 1,000-folded diluted Cy5-labeled annexin V and 2.5 µg/mL PI, and preincubated at 20°C for 5 minutes or at 4°C for 10 minutes. The cells were then stimulated with ionomycin, BzATP, or ATP at 20°C or 4°C and analyzed by flow cytometry using FACSCanto^{™}II. P2X7-dependent internalization of PtdCho was assayed by the internalization of NBD-PC as previously reported (Nature 468, 2010: 834-838). Specifically, 1.8×10⁶ cells were suspended in 300 µL of annexin buffer and preincubated at 4°C for 10 minutes. A portion of 250 µL of the cell suspension was mixed with an equal volume of annexin buffer containing 500 nM NBD-PC and 1 mM ATP and incubated at 4°C. 90 µL of this mixture was then mixed with 150 µL of annexin buffer containing 5 mg/mL fatty acid-free BSA, incubated on ice for 1 minute, and analyzed using FACSCanto^{™}II. Incorporation of YO-PRO-1 was similarly assayed by flow cytometry (J. Biol. Chem. 272, 1997: 5482-5486). Specifically, 1.3×10⁶ cells were preincubated at 4°C for 10 minutes in 650 µL of annexin buffer mixed with 650 µL of annexin buffer containing 4 µM YO-PRO-1, 500 nM SYTOX Blue, and 1 mM ATP, incubated at 4°C, and analyzed with FACSCanto^{™}II.

To monitor intracellular Ca²⁺, 7×10⁵ cells were incubated with 1.4 mL of 4 µM Fluo 4-AM in HEPES/NaCl buffer (10 mM HEPES-NaOH (pH 7.5) and 140 mM NaCl) at 25°C for 15 minutes, washed, incubated in 350 µL of HEPES/NaCl buffer at 25°C for 15 minutes, and cooled at 4°C for 10 minutes. Subsequently, 150 µL of the cell suspension was mixed with an equal volume of HEPES/NaCl buffer containing 5 mM CaCl₂, 500 nM SYTOX Blue, and 1 mM ATP, incubated at 4°C for 5 minutes, and analyzed by flow cytometry with FACSCanto^{™}II.

### Material and Method 4: Confocal Microscope

Cells stably expressing mP2X7k-EGFP, mEros-EGFP, and mEros-mCherry were washed with Hank's Balanced Salt Solution supplemented with 2% FCS (HBSS/FCS), and suspended in HBSS/FCS containing 5 µg/mL Hoechst 33342 or 5 µM DRAQ5. The cells were seeded in a glass bottom dish (Matsunami) and observed with an IX81 confocal fluorescence microscope (Olympus).

### Material and Method 5: RT-PCR for Mouse P2X7a and P2X7k

Some mouse tissues express two splice variants of P2X7, i.e., P2X7a and P2X7k, which use different exons 1 (J. Biol. Chem. 284, 2009: 25813-25822). To determine which variant is expressed in WR19L cells, total RNA was prepared from WR19L cells and resident peritoneal macrophages of C57BL/6J mice using an RNeasy Mini Kit (Qiagen), and reverse-transcribed using Superscript III Reverse Transcriptase (Thermo Fisher Scientific) or a High-Capacity RNA-to-cDNA kit (Thermo Fisher Scientific). Complementary DNA was subjected to PCR using PrimeSTAR GXL DNA Polymerase (Takara Bio) and the following primers: specific forward primers, 5'- TTTTTAATTAAGCCACCATGCCGGCTTGCTGCAGCTG-3' (SEQ ID NO: 6) on exon 1 for mP2X7a and 5'-TTTTTAATTAAGCCACCATGCTGCCCGTGAGCCAC-3' (SEQ ID NO: 7) on exon 1 for mP2X7k; and a common reverse primer, 5'-AAAGAATTCGTAGGGATACTTGAAGCCAC-3' (SEQ ID NO: 8) on exon 13.

### Material and Method 6: Genome-wide CRISPR Screening

Genome-wide CRISPR screening was performed according to a previous report (Science 343, 2014: 84-87, Nat. Protoc. 12, 2017: 828-863). Specifically, TMEM16F^{-/-}P2X7^{-/-}(DKO) WR19L cells established by a CRISPR-Cas9 system were stably transformed with pNEF-BOS-mP2X7k to generate DKO-WR19L-mP2X7k cells. A lentivirus carrying Cas9-FLAG was prepared by transfecting HEK293T cells with lentiCas9-Blast, pCAG-HIVgp, and pCMV-VSV-G-RSV-rev, and was used to infect the DKO-WR19L-mP2X7k cells. Transformants were selected in the presence of 10 µg/mL blasticidin S, and a clone expressing Cas9-FLAG was identified by Western blotting with anti-FLAG. A lentivirus for an sgRNA library was prepared by transfecting HEK293T cells (1×10⁷) with 17 µg of GeCKO v2 library (A+B) DNA, 8 µg of pCAG-HIVgp, and 5 µg of pCMV-VSV-G-RSV-rev, followed by concentration at 4°C by centrifugation at 6000×g for 16 hours. This virus was used to infect the DKO-WR19L-mP2X7k/Cas9 cells at an MOI of 0.3. Specifically, 2×10⁷ cells in a 48-well plate at 4×10⁵ cells/well were spin-infected at 700×g at 30°C for 1 hour in the presence of 10 µg/mL polybrene. The cells were then cultured in a medium containing 1 µg/mL puromycin and 800 µg/mL G418 for 4 days, with one passage with 3-fold dilution. The cells were then cultured without antibiotics for 2 days, during which the cells were passaged once with 4-fold dilution.

The resulting DKO-WR19L-mP2X7k/Cas9/GeCKO cells (3×10⁷) were washed with PBS, resuspended in 2 mL of annexin buffer, and cooled at 4°C for 10 minutes. Subsequently, 1 mL of cooled annexin buffer containing 180 µM BzATP was added to the cells, and the mixture was incubated at 4°C for 5 minutes. After 15-fold dilution with cooled annexin buffer, the cells were collected by centrifugation at 300×g at 4°C for 3 minutes, suspended in 3 mL of annexin buffer containing 1,000-fold diluted Cy5-labeled annexin V and 2.5 µg/ml PI, and subjected to cell sorting with FACSAria^{™}II. A cell population with the lowest annexin V signal (about 0.8%) was collected and cultured in the presence of 800 µg/mL G418 for 3 days and in the absence of G418 for 2 days. This procedure (stimulation with BzATP, sorting of cells expressing low levels of PtdSer, and proliferation) was performed three times.

Genomic DNA was prepared from the third sorted cells using a QIAamp DNA Mini Kit (Qiagen), and a portion of the integrated lentiviral DNA carrying the sgRNA sequence was amplified by PCR using PrimeSTAR GXL DNA Polymerase and the following primers. The primer sequences are as follows: 5'-AATGGACTATCATATGCTTACCGTAACTTGAAAGTATTTCG-3' (SEQ ID NO: 9), and '-CTTTAGTTTGTATGTCTGTTGCTATTATGTCTACTATTCTTTCC-3' (SEQ ID NO: 10). The PCR product was then subjected to PCR using PrimeSTAR HS DNA Polymerase (Takara Bio) to attach adaptor sequences for next-generation sequencing (NGS) using a mixture of forward primers (NGS-Lib-Fwd-1-10) (Nat. Protoc. 12, 2017: 828-863) and a common reverse primer (5'-CAAGCAGAAGACGGCATACGAGATGTGACTGGAGTTCAGACGTGTGCTCTTCCGATCTTCTACTA TTCTTTCCCCTGCACTGT-3' (SEQ ID NO: 11)). The resulting PCR product was purified with a spin column (Promega), quantified with a Quant-iT PicoGreen dsDNA Assay Kit (Thermo Fisher Scientific), and analyzed by NGS with a MiSeq (Illumina) using a MiSeq reagent Kit v3 (Illumina). The obtained reads were assigned to the sgRNA sequences, and the abundance of each unique sgRNA was calculated using software custom-prepared by Amelieff Co.

### Material and Method 7: Preparation of Whole-cell Lysate and Membrane Fraction

A whole-cell lysate was prepared by rotating cells in 50 mM Tris-HCl buffer (pH 7.5) containing 1% NP-40, 150 mM NaCl, and a protease inhibitor cocktail (cOmplete, EDTA-free, Roche) at 4°C for 1 hour. The insoluble materials were removed by centrifugation at 20,000×g at 4°C for 20 minutes.

To prepare a membrane fraction from WR19L cells, 2.5×10⁷ cells were washed with PBS and then homogenized on ice in 2.7 mL of solution A (10 mM Tris-HCl (pH 7.5) and 1 mM (p-amidinophenyl)methanesulfonyl fluoride hydrochloride (p-APMSF)) using a Dounce homogenizer. After 2.7 mL of solution B (10 mM Tris-HCl (pH 7.5), 500 mM sucrose, 100 mM KCl, 10 mM MgCl₂, and 1 mM p-APMSF) was added to the homogenate, the nuclei and mitochondria were removed at 4°C by sequential centrifugation at 800×g for 10 minutes and at 8,000×g for 10 minutes. The membrane fraction was then precipitated by centrifugation at 100,000×g for 60 minutes and suspended in 600 µL of 20 mM Tris-HCl buffer (pH 7.5) containing 1% n-dodecyl-β-D-maltopyranoside (DDM), 50 mM KCl, 1 mM MgCl₂, 10% glycerol, 1 mM p-APMSF, and a protease inhibitor cocktail (cOmplete, EDTA-free). The suspension was homogenized by passing it through a 29G needle and solubilized by rotation at 4°C for 2 hours. The insoluble materials were removed by centrifugation at 20,000×g for 20 minutes, and the supernatant was used as a crude membrane fraction.

To prepare a membrane fraction from THP-1 cells, 1.3×10⁷ cells were treated with 120 ng/mL phorbol 12-myristate 13-acetate (PMA) for 3 days and cultured for another 3 days without PMA. After washing with PBS, the cells were collected with a cell scraper and suspended in 2.5 mL of solution A. The cells were lysed with an ultrasonic liquid processor (Q55, Qsonica). After 2.5 mL of solution B was added, a membrane fraction was prepared as described above, and solubilized in 200 µL of 50 mM Tris-HCl buffer (pH 7.5) containing 1% NP-40, 150 mM NaCl, and a protease inhibitor cocktail (cOmplete, EDTA-free).

### Material and Method 8. Immunoprecipitation, SDS-PAGE, BN-PAGE, and Western Blotting

To immunoprecipitate an mP2X7-mEros complex, 500 µL of a crude membrane lysate (60 µg of protein) from a WR19L cell transformant expressing mP2X7-FLAG and mEros-EGFP was incubated overnight with 10 µL (bed volume) of anti-FLAG M2 magnetic beads. After washing three times with 1 mL of 20 mM Tris-HCl buffer (pH 7.5) containing 0.05% DDM, 50 mM KCl, 1 mM MgCl₂, and 10% glycerol, proteins bound to the beads were eluted with 100 µL of 20 mM Tris-HCl buffer (pH 7.5) containing 150 µg/mL 3×FLAG peptide, 1% DDM, 50 mM KCl, 1 mM MgCl₂, and 10% glycerol.

For SDS-PAGE, a whole-cell lysate or crude membrane lysate was incubated in SDS sample buffer (62.5 mM Tris-HCl (pH 6.8), 2% SDS, 10% glycerol, 2.5% β-mercaptoethanol, and 0.005% bromophenol blue) at room temperature for 1 hour and separated by electrophoresis on a 7.5% polyacrylamide gel (Nacalai Tesque, Inc.). Precision Plus Protein Dual Color Standards (Bio-Rad Laboratories, Inc.) were used as molecular weight markers. For BN-PAGE, a sample was mixed with NativePAGE (registered trademark) Sample Buffer (4×) and NativePAGE (registered trademark) 5%G-250 Sample Additive (20×) and loaded onto a NativePAGE (registered trademark) Novex 4-16% BisTris gel (Thermo Fisher Scientific). After electrophoresis at 150 V at 4°C for 35 minutes, the concentration of CBB G-250 in the running buffer was changed from 0.02 to 0.002%, and the sample was subjected to further electrophoresis successively at 150 V for 25 minutes, at 250 V for 30 minutes, and at 350 V for 20 minutes. NativeMark (registered trademark) Unstained Protein Standard (Thermo Fisher Scientific) was used as a molecular weight marker.

For Western blotting, immediately after SDS-PAGE or immediately after incubating the BN-PAGE gel in SDS-PAGE running buffer (25 mM Tris-HCl (pH 8.3), 192 mM glycine, and 0.1% SDS) at room temperature for 15 minutes, separated proteins in the gel were transferred to a PVDF membrane (Millipore). The membrane was blocked with 5% skim milk and probed with an HRP-labeled antibody, followed by detection with Immobilon Western Chemiluminescent HRP Substrate (Merck). As a loading control, proteins on the PVDF membrane were stained with CBB R-250.

### Material and Method 9: IL-1β ELISA

THP-1 cells were treated with PMA to promote their differentiation into macrophages, and ATP-induced IL-1β secretion was performed by a method slightly modified from the method described in a previous report (Immunity 15, 2001: 825-835, PLoS ONE 5, 2010: e8668). Specifically, THP-1 cells at a density of 2.5×10⁵ cells/well in a 24-well plate were cultured in RPMI 1640 containing 10% FCS and 120 ng/mL PMA for 3 days. The PMA was removed, and the cells were cultured in RPMI 1640-10% FCS for 2 days and further in the same medium containing 100 ng/mL LPS overnight. The cells were then stimulated with 1 to 3 mM ATP in RPMI 1640-10% FCS for 5 hours. The culture liquid was centrifuged at 20,000×g at 4°C for 15 minutes, and IL-1β in the supernatant was quantified with an ELISA kit (Quantikine ELISA Human IL-1β/ IL-1F2, R&D Systems).

### Results

### Test Example 1: P2X7-dependent but TMEM16F-independent PtdSer Exposure

The assays in this Test Example were performed according to the "Material and Method 3" section above. Fig. 1 shows the results of this Test Example. The outline, conditions, and the like of the specific assay shown in Fig. 1A are as follows. After preincubation at 25°C for 15 minutes in the presence or absence of 25 µM BAPTA-AM, WR19L cells were left untreated or stimulated with 3 µM ionomycin or 300 µM BzATP at 20°C for 5 minutes. The cells were then stained with Cy5-labeled annexin V in the presence of 2.5 µg/mL PI and analyzed by flow cytometry. Annexin V staining in the PI-negative population is shown. The outline, conditions, and the like of the specific assay shown in Fig. 1B are as follows. Wild-type, TMEM16F^{-/-} (16F^{-/-}), P2X7^{-/-}, and 16F^{-/-}P2X7^{-/-} (DKO) WR19L cells were stimulated with ionomycin or BzATP as described above, stained with annexin V and PI, and analyzed by flow cytometry.

A previous report (Nat. Commu. 6, 2015: 6245-6210) reported that P2X7-induced PtdSer exposure is mediated by a Ca²⁺-dependent scramblase TMEM16F. Thus, this possibility was examined using a mouse cell line derived from WR19L T-cell leukemia. WR19L cells stimulated with 3 µM ionomycin at 20°C exposed PtdSer within 5 minutes (Fig. 1A and Fig. 1B). As previously reported (J. Biol. Chem. 288, 2013: 13305-13316), this PtdSer exposure was completely suppressed by chelating intracellular Ca²⁺ with 25 µM BAPTA-AM (Fig. 1A), and this phenomenon was not observed in TMEM16F^{-/-} (16F^{-/-}) WR19L cells (Fig. 1B) . Treatment of WR19L cells with 300 µM BzATP (Blood 97, 2001: 587-600), an agonist for P2X7, at 20°C for 5 minutes strongly increased PtdSer exposure (Fig. 1A). As expected, BzATP did not induce PtdSer exposure in P2X7^{-/-}WR19L cells (Fig. 1B). However, in contrast to the proposal in a previous report (Nat. Commu. 6, 2015: 6245-6210), the TMEM16F defective mutation had little effect on BzATP-induced PtdSer exposure (Fig. 1B). Furthermore, preincubation of WR19L cells with BAPTA-AM had no effect on BzATP-induced PtdSer exposure (Fig. 1A), indicating that other TMEM16 family members with Ca²⁺-dependent scrambling activity are probably not involved in this process. From these results, the present inventors concluded that P2X7-induced PtdSer exposure in WR19L cells proceeds independently of TMEM16F-mediated phospholipid scrambling.

### Test Example 2: Screening for Molecules That Support P2X7-mediated PtdSer Exposure

The assays in this Test Example were performed according to the "Material and Method 3," "Material and Method 5," and "Material and Method 6" sections above. Fig. 2 shows the results of this Test Example. The outline, conditions, and the like of the specific assay shown in Fig. 2A are as follows. Transcripts of P2X7a and P2X7k isoforms were detected by RT-PCR in RNA from mouse resident peritoneal macrophages (rpMac) and WR19L cells. The P2X7a and P2X7k isoforms were generated by using different first exons (exon 1 and exon 1'). The forward primers on the first exons were specific for each isoform, whereas the reverse primers on exon 13 were in common. The outline, conditions, and the like of the specific assay shown in Fig. 2B are as follows. 16F^{-/-}, DKO, and DKO-mP2X7k cells were stained with Alexa488-anti-P2X7 Ab and analyzed by flow cytometry. In addition, 16F^{-/-}, DKO, and DKO-mP2X7k were treated with the indicated concentrations of BzATP at 20°C for 5 minutes, stained with Cy5-labeled annexin V, and analyzed by flow cytometry. The experiment was performed in triplicate, and the average percentage of annexin V-positive cells in the PI-negative population was plotted with SD. The outline, conditions, and the like of the specific assay shown in Fig. 2C are as follows. In the first step, DKO-WR19L-mP2X7k cells were transformed with Cas9 and CRISPR library, and a population of cells with reduced activity for BzATP-induced PtdSer exposure was sorted three times. In the second step, DNA from these sorted cells was subjected to deep sequencing, and the read sequences were processed to determine the abundance of each sgRNA. The original cells and the cells after the third sorting were left unstimulated or stimulated with BzATP and then analyzed by flow cytometry.

There are several alternatively spliced forms of mouse (m)P2X7 mRNA (Biochem. Biophys. Res. Commun. 332, 2005: 17-27), two of which (P2X7a and P2X7k) are expressed in the thymus and spleen (J. Biol. Chem. 284, 2009: 25813-25822, J. Cell Sci. 125, 2012: 3776-3789). To examine which splice variant is expressed in WR19L cells, their RNA was analyzed by P2X7a and P2X7k isoform-specific RT-PCR. As previously reported, the mouse macrophages expressed only P2X7a mRNA, whereas the WR19L cells mainly expressed the P2X7k isoform (Fig. 2A). To confirm the contribution of mP2X7k to BzATP-induced PtdSer exposure, TMEM16F^{- /-}P2X7^{-/-}(DKO)-WR19L cells were established using a CRISPR-Cas9 system. As expected, the DKO-WR19L cells did not respond to Caionomycin or BzATP (Fig. 1B). Transformation of the DKO-WR19L cells with mP2X7k (DKO-mP2X7k) resulted in high expression of mP2X7k on their cell surface (Fig. 2B) and strongly sensitized the cells to BzATP-induced PtdSer exposure. That is, the concentration of BzATP that is three times lower than that for the wild-type cells was sufficient to activate the mP2X7k-transformed cells to expose PtdSer at 20°C for 5 minutes in the absence of TMEM16F, confirming that TMEM16F was not required for P2X7-mediated PtdSer exposure.

To identify molecules required for P2X7-mediated PtdSer exposure, DKO-mP2X7k cells were subjected to CRISPR screening (Science 343, 2014: 84-87) (Fig. 2C). In this screening, clones of mP2X7k transformants that strongly and universally exposed PtdSer in response to 60 µM BzATP at 4°C for 5 minutes were identified by limiting dilution and cell sorting. The cloned cells were transformed with Cas9 and infected with a lentivirus carrying a GeCKO (genome-scale CRISPR-Cas9 knockout) library at an MOI (multiplicity of infection) of 0.3 to achieve less than 1 sgRNA-carrying lentivirus per host cell. The cells were then stimulated with 60 µM BzATP at 4°C for 5 minutes, and a 0.8% population that markedly lost the ability to expose PtdSer was isolated by cell sorting (Fig. 2C). This sorting of cells that lost the ability to expose PtdSer in response to BzATP was repeated two more times. Flow cytometry analysis showed that after the third sorting, some cell populations almost completely lost the ability to expose PtdSer in response to BzATP (Fig. 2C). Deep sequencing analysis of the chromosomal DNA of the sorted cell population showed that the sgRNA (CYBC1) for Eros was most enriched, suggesting that Eros is required for P2X7-mediated PtdSer exposure.

### Test Example 3. Requirement of Eros for P2X7-mediated Process

The assays in this Test Example were performed according to the "Material and Method 3" and "Material and Method 9" sections above. Fig. 3 shows the results of this Test Example. The outline, conditions, and the like of the specific assay shown in Fig. 3A are as follows. DKO-mP2X7k, TKO-mP2X7k, and TKO-mP2X7k/mEros cells were stimulated with 60 µM BzATP or 500 µM ATP at 4°C for 5 minutes, stained with annexin V, and analyzed by flow cytometry. The outline, conditions, and the like of the specific assay shown in Fig. 3B and Fig. 3C are as follows. DKO-WR19L, DKO-WR19L-mP2X7k, TKO-WR19L-mP2X7k, and TKO-WR19L-mP2X7k/mEros cells were stimulated with 500 µM ATP at 4°C for the specified time in the presence of 250 nM NBDPC(B) or 2 µM YO-PRO-1(C). The mean fluorescence intensity (MFI) of BSA-nonextractable NBD-PC or incorporated YO-PRO-1 in the SYTOX Blue-negative population was determined. The experiment was performed in triplicate, and the average MFI or its relative value (relative fluorescence intensity (RFI)) was plotted with SD. The outline, conditions, and the like of the specific assay shown in Fig. 3D are as follows. DKO, DKO-mP2X7k, TKO-mP2X7k, and TKO-mP2X7k/mEros cells were loaded with Fluo 4-AM and stimulated with 500 µM ATP at 4°C for 5 minutes. Fluo 4 fluorescence profiles in the SYTOX Blue-negative population are shown. The outline, conditions, and the like of the specific assay shown in Fig. 3E are as follows. After being treated with PMA, wild-type THP-1, Eros^{-/-}, Eros^{-/-} hEros, P2X7^{-/-}, and P2X7^{-/-}hP2X7a cells were incubated with 100 ng/mL LPS at 37°C overnight and cultured in the presence of the indicated concentrations of ATP for 5 hours. IL-1β in the culture medium was measured by ELISA. The experiment was performed in triplicate, and the average values were shown with SD. The outline, conditions, and the like of the specific assay shown in Fig. 3F are as follows. BMDMs from wild-type and Eros^{-/-} mice were incubated in the presence or absence of 100 ng/ml LPS at 37°C for 3 hours and cultured in the presence of the indicated concentrations of ATP for 6 hours. IL-1β in the culture medium was measured by ELISA. The experiment was performed in triplicate, and the average values were shown with SD (bars).

To confirm that Eros is involved in P2X7-mediated PtdSer exposure, Eros in DKO-mP2X7k was knocked out by a CRISPR-Cas9 system. The ability of the obtained cell line (TKO-mP2X7k) to expose PtdSer in response to BzATP or ATP was markedly impaired, and this impairment was completely rescued by transforming the cells with mEros (TKO-mP2X7k/mEros) (Fig. 3A). P2X7 mediates not only PtdSer exposure, but also ATP-induced phospholipid scrambling, dye uptake, Ca²⁺ influx, and IL-1β production. Accordingly, stimulation of DKO-mP2X7k with ATP promoted phospholipid scrambling, which was assayed by incorporation of NBD-PC (Fig. 3B). This activity was not observed in TKO-mP2X7k cells and was rescued by transforming these cells with Eros. Similarly, ATP promoted the incorporation of YO-PRO-1 in DKO-mP2X7k cells. This effect was decreased in TKO-mP2X7k, and transformation of TKO cells with mEros together with mP2X7 strongly increased the incorporation of YO-PRO-1. Furthermore, DKO cells did not respond to ATP for Ca²⁺ internalization, but DKO-mP2X7k cells responded well to ATP, and intracellular Ca²⁺ detected by a Fluo-4 Ca²⁺ sensor increased within 5 minutes after ATP treatment (Fig. 3D). This response was not observed in TKO-mP2X7k cells, but was rescued by expressing mEros in TKO-mP2X7k/mEros cells. The amino acid sequence of human P2X7 has 81.3% identity to the amino acid sequence of mouse P2X7. As previously reported (Immunity 15, 2001: 825-835), human THP-1-derived macrophages stimulated with a lipopolysaccharide (LPS) produced IL-1β in response to ATP in a dose-dependent manner (Fig. 3E). To examine the contribution of Eros in this process, the Eros and P2X7 genes in THP-1 cells were individually knocked out by a CRISPR/Cas9 system. As shown in Fig. 3E, not only the P2X7-null mutation, but also the Eros-null mutation completely blocked IL-1β production, and these defects were rescued by expressing human (h)P2X7 or hEros, respectively. Subsequently, BMDMs were prepared from wild-type and Eros^{-/-} mice. Consistent with previous reports, LPS-primed, wild-type BMDMs produced IL-1β in response to ATP (Fig. 3F). In contrast, Eros^{-/-} BMDMs almost completely lost the ability to produce IL-1β in response to ATP (Fig. 3F). The results show that the production of IL-1β can be suppressed by suppressing Eros. The effect exhibited by suppression of IL-1β is widely known and described, for example, in BioDrugs (2017) 31: 207-221.

From these results, it was concluded that various P2X7-mediated biological processes in mouse and human cells require Eros.

### Test Example 4: Chaperone-like Activity of Eros for P2X7 Expression

The assays in this Test Example were performed according to the "Material and Method 3," "Material and Method 4," and "Material and Method 8" sections above. Fig. 4 shows the results of this Test Example. The outline, conditions, and the like of the specific assay shown in Fig. 4A are as follows. Cell lysates (1.5 µg of protein) from DKO (lane 1), DKO-mP2X7k-FLAG (lane 2), TKO-mP2X7k-FLAG (lane 3), and TKO-mP2X7k-FLAG/mEros cells (lane 4) were separated, analyzed by SDS-PAGE, and analyzed by Western blotting using anti-FLAG Ab. The membrane was stained with CBB R-250. Arrowheads indicate trimeric and monomeric forms of mP2X7. The outline, conditions, and the like of the specific assay shown in Fig. 4B are as follows. Wild-type (lane 1), Eros^{-/-}(lane 2), Eros^{-/-} hEros-HA (lane 3), P2X7^{-/-} (lane 4), and P2X7^{-/-}hP2X7a-FLAG cells (lane 5) were treated with PMA. Crude membranes (7.5 µg of protein) were solubilized with 1% NP-40, separated by SDS-PAGE, and analyzed by Western blotting using anti-P2X7 Ab. The membrane was probed with anti-Na⁺/K⁺-ATPase, anti-HA, or anti-FLAG Ab. Arrowheads indicate trimeric and monomeric forms of hP2X7. The outline, conditions, and the like of the specific assay shown in Fig. 4C are as follows. DKO, DKO-mP2X7k, TKO-mP2X7k, and TKO-mP2X7k/mEros cells were stained with Alexa 488-anti-mP2X7 Ab. The Alexa 488 staining profiles in the SYTOX Blue-negative population are shown. The outline, conditions, and the like of the specific assay shown in Fig. 4D and Fig. 4E are as follows. TKO WR19L cells were transformed with mP2X7k-EGFP and mEros, mP2X7k and mEros-EGFP(D), or mP2X7k-EGFP and mEros-mCherry(E), and observed with a confocal microscope in the presence of 5 µM DRAQ5 or 5 µg Hoechst 33342. EGFP, mCherry, DRAQ5, and Hoechst signals are shown in green, red, magenta, and blue, respectively. The outline, conditions, and the like of the specific assay shown in Fig. 4F are as follows. Crude membrane fractions (1.2 µg of protein) from DKO-mP2X7k-FLAG (lane 1), TKO-mP2X7k-FLAG (lane 2), and TKO-mP2X7k-FLAG/mEros-EGFP cells (lane 3) were solubilized with 1% DDM, separated by BN-PAGE, and analyzed by Western blotting using anti-FLAG (left panel) or anti-GFP Ab (right panel). The outline, conditions, and the like of the specific assay shown in Fig. 4G are as follows. Crude membranes (60 µg of protein) from DKO-mP2X7k-FLAG or TKO-mP2X7k-FLAG/mEros-EGFP cells were solubilized with 1% DDM. The FLAG-tagged proteins were immunoprecipitated with anti-FLAG M2 magnetic beads and eluted with 3×FLAG peptide. The crude membrane lysates (1.5 µg of protein) (input) and 1/16 of the eluates (IP) were separated by BN-PAGE and analyzed by Western blotting using anti-GFP (top panel) or anti-FLAG Ab (bottom panel).

Eros was recently shown to be required for the stable expression of NADPH oxidase subunits gp91phox and p22phox by acting as a chaperone (J. Exp. Med. 214, 2017: 1111-1128). To examine whether Eros acts as a chaperone for P2X7, FLAG-tagged mP2X7k was introduced into DKO, TKO, and TKO-mEros cells, and the mP2X7k expression was evaluated by Western blotting using an anti-FLAG antibody. As shown in Fig. 4A, mP2X7k was stably expressed in DKO cells. However, its expression was markedly decreased in TKO cells, and this decrease was rescued by transforming the cells with mEros. Similar results were obtained for endogenous hP2X7 in THP-1 cells. Western blotting with anti-P2X7 Ab detected bands of 75 kDa and 200 kDa in the membrane fraction from the wild type, but not in P2X7^{-/-}THP-1 cells. The intensity of these bands was increased by transforming the cells with hP2X7-FLAG (Fig. 4B), indicating that these bands corresponded to monomeric and trimeric forms of hP2X7. The expression of hP2X7 was severely decreased by null mutation in Eros, and this decrease was completely rescued by expressing hEros.

The requirement of Eros for cell surface expression of P2X7 was then examined using WR19L cells. Flow cytometry analysis using anti-mP2X7 Ab showed that mP2X7 was highly expressed in DKO-mP2X7k cells. In contrast, mP2X7 on the surface of TKO-mP2X7k cells was markedly decreased, but it was completely rescued in TKO-mP2X7k/mEros cells. To examine the localization of mP2X7 and mEros, mP2X7k and mEros were fused with EGFP and expressed in TKO cells together with mEros-HA or mP2X7-FLAG, respectively. Observation with a confocal microscope showed that a substantial portion of mP2X7k localized to the plasma membrane (Fig. 4D). On the other hand, consistent with previous reports, mEros-EGFP in TKO-mP2X7k cells was observed exclusively within the cells (probably at the endoplasmic reticulum). When TKO cells were transformed to coexpress mP2X7k-EGFP and mEros-mCherry, the intracellular EGFP signal colocalized with the mCherry signal (Fig. 4E). In contrast, the EGFP signal on the plasma membrane did not colocalize with mCherry, suggesting that mEros assisted the folding or maturation of mP2X7 at the endoplasmic reticulum and that the mature mP2X7 moved to the plasma membrane. To test this possibility, DKO, TKO, and TKO-mEros-EGFP cells were transformed with FLAG-tagged mP2X7k, and their membrane fractions were analyzed by Blue native (BN)-PAGE. As shown in Fig. 4F, Western blotting using an anti-FLAG antibody showed two bands at around 800 kDa and 550 kDa in the DKO and TKO22 mEros, but not in the TKO cells. The lower band at 550 kDa appeared to correspond to the trimeric form of mP2X7k, as previously reported. Anti-GFP Ab for mEros-EGFP detected two bands, of which the upper band was similar to the upper band observed with the anti-FLAG Ab for mP2X7k. The membrane fractions were then subjected to immunoprecipitation using anti-FLAG beads for mP2X7k-FLAG. As shown in Fig. 4G, the precipitates carried the upper large complex, which was recognized not only with anti-FLAG for mP2X7k, but also with anti-GFP for mEros. These results showed that the large band of 800 kDa was a complex between mP2X7k and mEros in the endoplasmic reticulum. Mature mP2X7k with an apparent Mr (relative molecular mass) of 550 kDa was present on the plasma membrane, whereas mEros, free or complexed with other proteins, was present in the endoplasmic reticulum. From these results, it was concluded that mEros transiently interacts with mP2X7 in the endoplasmic reticulum and assists its folding. It was believed that mP2X7 then moves to the plasma membrane, where it is present as a homotrimeric complex.

## Claims

1. A P2X7 receptor expression modulator comprising at least one member selected from the group consisting of an Eros (essential for reactive oxygen species) expression modulator and a functional modulator of Eros.

2. The modulator according to claim 1, which comprises at least one member selected from the group consisting of an Eros expression suppressor and an Eros function suppressor, and is for suppressing P2X7 receptor expression.

3. The modulator according to claim 2, which comprises an Eros expression suppressor.

4. The modulator according to claim 3, wherein the Eros expression suppressor comprises a polynucleotide.

5. The modulator according to claim 3 or 4, wherein the Eros expression suppressor comprises at least one member selected from the group consisting of an Eros-specific siRNA, an Eros-specific miRNA, an Eros-specific antisense nucleic acid, and an expression cassette thereof, and an Eros gene-editing agent.

6. The modulator according to any one of claims 2 to 5, which is for preventing or ameliorating at least one member selected from the group consisting of inflammation and pain.

7. The modulator according to claim 1, which comprises at least one member selected from the group consisting of an Eros expression promoter and an Eros function promoter, and is for promoting P2X7 receptor expression.

8. The modulator according to claim 7, which comprises an Eros expression promoter.

9. The modulator according to claim 8, wherein the Eros expression promoter comprises an Eros expression cassette.

10. The modulator according to any one of claims 7 to 9, which is for promoting differentiation and/or maintenance of a memory T cell.

11. The modulator according to any one of claims 1 to 10, which is a medicament, a reagent, or a food composition.

12. A T cell into which the modulator according to any one of claims 7 to 10 has been introduced.

13. The T cell according to claim 12, which is capable of modulating Eros expression and/or Eros function so that the Eros expression and/or Eros function is transiently enhanced.

14. The T cell according to claim 12 or 13, into which the modulator according to any one of claims 2 to 5 has been further introduced.

15. The T cell according to claim 14, which is capable of modulating Eros expression and/or Eros function so that the Eros expression and/or Eros function is suppressed, after transiently enhancing the Eros expression and/or Eros function.


SEQUENCE LISTING

<110>  Osaka University

<120>  P2X7 receptor expression regulator

<130>  G20200007(PCT)

<150>  JP2019-098049
<151>  2019-05-24

<160>  15

<170>  PatentIn version 3.5

<210>  1
<211>  20
<212>  DNA
<213>  Artificial Sequence

<220>
<223>  mTMEM16F sgRNA

<400>  1
ggatgaagtc gattcgcctc                                                   20


<210>  2
<211>  20
<212>  DNA
<213>  Artificial Sequence

<220>
<223>  mP2X7 sgRNA

<400>  2
tgagcgataa gctgtaccag                                                   20


<210>  3
<211>  20
<212>  DNA
<213>  Artificial Sequence

<220>
<223>  mEros sgRNA

<400>  3
atttgtggct gtacagaact                                                   20


<210>  4
<211>  20
<212>  DNA
<213>  Artificial Sequence

<220>
<223>  hP2X7 sgRNA

<400>  4
tgatgacagg ctctttccgc                                                   20

<210>  5
<211>  20
<212>  DNA
<213>  Artificial Sequence

<220>
<223>  hEros

<400>  5
tggaagctct tctacgtcac                                                    20


<210>  6
<211>  37
<212>  DNA
<213>  Artificial Sequence

<220>
<223>  mP2X7a F primer

<400>  6
tttttaatta agccaccatg ccggcttgct gcagctg                                 37


<210>  7
<211>  35
<212>  DNA
<213>  Artificial Sequence

<220>
<223>  mP2X7k F primer

<400>  7
tttttaatta agccaccatg ctgcccgtga gccac                                   35


<210>  8
<211>  29
<212>  DNA
<213>  Artificial Sequence

<220>
<223>  mP2X7 common R primer

<400>  8
aaagaattcg tagggatact tgaagccac                                          29


<210>  9
<211>  41
<212>  DNA
<213>  Artificial Sequence

<220>
<223>  primer 1 for material and method 6

<400>  9
aatggactat catatgctta ccgtaacttg aaagtatttc g                            41


<210>  10
<211>  44
<212>  DNA

<213>  Artificial Sequence

<220>
<223>  primer 2 for material and method 6

<400>  10
ctttagtttg tatgtctgtt gctattatgt ctactattct ttcc                        44


<210>  11
<211>  83
<212>  DNA
<213>  Artificial Sequence

<220>
<223>  primer 3 for material and method 6

<400>  11
caagcagaag acggcatacg agatgtgact ggagttcaga cgtgtgctct tccgatcttc       60

tactattctt tcccctgcac tgt                                               83


<210>  12
<211>  187
<212>  PRT
<213>  Homo sapiens

<400>  12

Met Tyr Leu Gln Val Glu Thr Arg Thr Ser Ser Arg Leu His Leu Lys
1               5                   10                  15


Arg Ala Pro Gly Ile Arg Ser Trp Ser Leu Leu Val Gly Ile Leu Ser
            20                  25                  30


Ile Gly Leu Ala Ala Ala Tyr Tyr Ser Gly Asp Ser Leu Gly Trp Lys
        35                  40                  45


Leu Phe Tyr Val Thr Gly Cys Leu Phe Val Ala Val Gln Asn Leu Glu
    50                  55                  60


Asp Trp Glu Glu Ala Ile Phe Asp Lys Ser Thr Gly Lys Val Val Leu
65                  70                  75                  80


Lys Thr Phe Ser Leu Tyr Lys Lys Leu Leu Thr Leu Phe Arg Ala Gly
                85                  90                  95


His Asp Gln Val Val Val Leu Leu His Asp Val Arg Asp Val Ser Val
            100                 105                 110


Glu Glu Glu Lys Val Arg Tyr Phe Gly Lys Gly Tyr Met Val Val Leu
        115                 120                 125


Arg Leu Ala Thr Gly Phe Ser His Pro Leu Thr Gln Ser Ala Val Met

130                 135                 140

Gly His Arg Ser Asp Val Glu Ala Ile Ala Lys Leu Ile Thr Ser Phe
145                 150                 155                 160

Leu Glu Leu His Cys Leu Glu Ser Pro Thr Glu Leu Ser Gln Ser Ser
                165                 170                 175

Asp Ser Glu Ala Gly Asp Pro Ala Ser Gln Ser
            180                 185

<210>  13
<211>  187
<212>  PRT
<213>  Mus musculus

<400>  13

Met Tyr Met Gln Val Glu Thr Arg Thr Ser Thr Arg Leu His Leu Lys
1               5                   10                  15

Arg Ala Pro Gly Ile Arg Ser Trp Ser Leu Leu Val Gly Ile Leu Ser
            20                  25                  30

Thr Gly Leu Ala Ala Ala Tyr Tyr Ser Gly Asp Ser Leu Gly Trp Lys
        35                  40                  45

Leu Phe Tyr Val Thr Gly Cys Leu Phe Val Ala Val Gln Asn Leu Glu
    50                  55                  60

Asp Trp Glu Glu Ala Ile Phe Asn Lys Asn Thr Gly Lys Val Ile Leu
65                  70                  75                  80

Lys Thr Phe Ser Leu Tyr Lys Lys Leu Leu Thr Leu Leu Arg Ala Gly
                85                  90                  95

His Asp Gln Val Val Val Leu Leu Lys Asp Ile Gln Asp Val Asn Val
            100                 105                 110

Glu Glu Glu Lys Val Arg Tyr Phe Gly Lys Gly Tyr Met Val Val Leu
        115                 120                 125

Arg Phe Ala Thr Gly Phe Ser His Pro Leu Thr Gln Ser Ala Val Met
    130                 135                 140

Gly Arg Arg Ser Asp Val Glu Ala Ile Ala Lys Leu Ile Thr Ser Phe
145                 150                 155                 160

Leu Glu Leu His Arg Leu Glu Ser Pro Ser Glu Arg Ser Gln Ser Ser

165                 170                 175

Asp Ser Glu Pro Asp Gly Pro Gly Gly Gln Ser
            180                 185

<210>  14
<211>  2217
<212>  DNA
<213>  Homo sapiens

<400>  14
ccaatcccgg aagcgcgcgc ggccccgccc ccgaaagtcg gctcggctcc gctccaatcc      60

cggaagtgcg cgcggccccg cccccgccgg ttcgcgtctc tctgctgcgg cgcggggacc     120

gctgtgctct cggaaacggg gtctctgggg gatccagtga cgtgcccaac cacagagacc     180

agacccctcc tcctgtagag agtggtgctg ccctctcggg atgtacctgc aggtggagac     240

ccgcaccagc tcccgcctcc atctgaagag ggctccaggc atccggtcct ggtccctgct     300

ggttggaatc ttgtcgattg gcctggctgc tgcctactac agcggagata gcctgggctg     360

gaagctcttc tacgtcacag gctgcctgtt tgtggctgtg cagaacttgg aggactggga     420

ggaagccatc ttcgacaaga gcacagggaa ggttgttttg aagacgttca gcctctacaa     480

gaagctgctg actcttttca gagctggcca cgaccaggtg gtggtcctgc tccatgatgt     540

ccgtgatgtg agcgtggagg aggagaaggt ccggtacttc gggaaaggct acatggtggt     600

gctccggctt gcgacgggct tctcccaccc cctcacgcag agtgcagtca tgggccaccg     660

cagtgatgtg gaagccatcg ccaagctcat caccagcttc ctggagctgc actgccttga     720

gagccccaca gagctgtctc agagcagcga cagtgaggcc ggtgaccctg caagccagag     780

ctgacagccc cactgtgcct gagcccgtgc accgcccaca ggacccatgg cacattcccg     840

gtgtgcctga gcccgtgcac cgcccacagg acccgtggca cattcccggt gtgcctgagc     900

ccgtgcaccg cccacaggac ccgtggcaca ttcccggtgt gcctgagccc gtgcaccgcc     960

cacaggaccc gtggcacatt cccggtgtgc ctgagcccgt gcaccgccca caggacccgt    1020

ggccttggct tcagttggtg cctccagccg agttggccta ttgcctgctc atgctgctgc    1080

ttgcacactg catgaaacag caggccagac caggacatcc agactttctc catcgtgagg    1140

cctgggcctg cctttctgca gccggaggtc tcgccagccc tggactcctg ctttgggcca    1200

cagcaagacc tcgggcgagt ggagaggcgg ggccaggccg ggcccttgtg ggtgctgatg    1260

ctgcatgttg tccccgacac agcgtcctct ccctggtgga catccccagc ggtcaggtgc    1320

ttccccaagg gcagtgaggg gtgaacatcc agggcctacc tggctgtgca cgctgcagcc    1380

acactgtgga agctgcccct ccccgaggac ccgcctccct tgctgatgcc aggatctcgg    1440

cgcatagacc actctgcccc agcggtcgtc acagaaaggt ctctctgttc ctcacactca    1500

gcttcagcat aagctgtgag gccagaaaaa aggtcagctc ttctagtatc gtgcagtgct 1560

taaaaaccgg gagctccagc cgggcgcagt ggttcatgcc agtaatccca gcactttcgg 1620

aggccgaggt gggaggattg cttgaggcca ggagttcaag accagcctgg gcaacacagc 1680

aagatcctgt ctttgtaaaa aaactaacca aacaggaaaa actgggagat tttctgcaga 1740

aattgagttc cagcctctct cgaacctggg aagacctggc aggagggggc tgggctctcg 1800

gctacagact tctccccacc ccgtaggagc tgaacgccaa ccatcctgac ccgccagtgc 1860

tcttggtctc ctgagtgtac ccaggtcctc ccaggtgcgg tgtgcaccga gcgcgcctgg 1920

cctgatgccc tggcctgtga gctggggact cctgggccct gtgagcccct aggcggcagg 1980

cccaggaatg gctgggtagg acagggaaca cctttgcccc acgtctggct gtgacctcgg 2040

tgaaagccga caggagagag atgggaccct cctcctcagt agtggctgcc agtccctcgt 2100

tgcaggacag ggtcatcata accataaata acccttcacg tgtcacctgc agcttccact 2160

ctttatttcc aaagaatcag tgtcacacat gcagatcaca aaaaaaaaaa aaaaaaa 2217

<210> 15
<211> 2483
<212> DNA
<213> Mus musculus

<400> 15
   agctcttatc ccggaagtct gcggaacaga agtccggaag ttcgaggggc tgttctctcc 60
   cactcattct cctacaagtc cctcgttgtc cgcataggtg cgtgtcctac aacttttcgg 120
   gttccgaccc gccgagccct gtgcgggtca tcgcttgcgg atctggacca ggcgcaaggc 180
   aaattgagga agccgggcgt gtgcactccg gaggaggagc cagcgtaacg ggccgctagt 240
   ggagcttgtt ggtaactgaa gccagaggga ggcttcagtt gtgtttttcc aagcctggtc 300
   ggctgtgcag tagagcccag gttccaagaa tgtatatgca ggtggagaca cgcaccagca 360
   cccgcctcca tctgaagagg gctccaggca tccggtcttg gtccctgctg gtcggaatat 420
   tatccactgg cctggctgct gcctattaca gtggagatag tctgggctgg aagctcttct 480
   atgtcacagg ctgcctattt gtggctgtac agaacttgga agactgggag gaggccattt 540
   tcaacaagaa cactgggaag gtcatcttaa aaacattcag cctctacaag aagctgctga 600
   ctcttctcag agcgggccac gatcaagtgg tggttctgtt gaaagacatc caggatgtga 660
   atgtggagga ggaaaaggtc cgttactttg ggaagggcta catggtggtg ctccggtttg 720
   caacaggctt ctcccaccct ctcactcaga gtgcagtcat gggccgacgc agtgatgtgg 780
   aagccattgc caaactcatc actagcttct tggagctgca ccgcctggag agcccctcag 840
   aacggtcaca gagcagcgac agtgaacctg atggccctgg tggccagagt tgacagtgtg 900
   gactggctgc aggtaaatgc catccacttc tgatgcatct ccaaccgact tgacaattgt 960
   aagggtacag gcagatcttg ggcacctgcc tgtggctcct tgggcagttc tactatgggc 1020
   cacctgcctt ttgcttgttt atgcttctac actttgcaga accttgggct agacctctgc 1080
   cacaacccag aaaggttgca tctatcagga tgcagggtgt ggatgctaca tctagggggg 1140
   gcaaggtctc agaaatgagt gccttctcca gagttacact gcagctctgg gttgggcagc 1200
   acagcctggt ctggataagc tcacccaagg gcaatctgaa gcagaaaatg ccaagctctc 1260
   ttcagcatag gcctagtgcc tgccttggag acaccttgcc actccaggct actgtaagca 1320
   agactccatt cctagtgagt gagacaccta tatgcacgtg ggcagacaca ctcctatgca 1380
   cagtgatgtc tgttccctct gaccatccct caagatggac actctcagtg gtcaggggct 1440
   tccctgaagg caactctgga tgctggacac ctgaccacag aactctcaga ggaatccgag 1500
   tgctgctgac tacccagttc cttcttagac catgtgtctg agagcccagc agtgttggag 1560
   cccagctgtc aggaatggaa ggctctccca acccagcctg gaaagaaggt gctagcagag 1620
   gaaagatcac ctctcctggc atcctgcagc ctttaagacc tgggggatcc cttacagaaa 1680
   cagtcttagc ctttcgagcc ttgaaaagac ctgaagaaaa caggcatgtg gagacacagc 1740
   ccaataaatc ccagtgttga gaactgcagc gggaagaacg ttcaaggcca gcctgggcca 1800
   tggcctcttc taagaaaagt tggtaccaac cactaatggt ccttttctgc tcttctagtc 1860
   tccttctggg tggactcaag ttgctcaaaa gtacattgtt tactaagttc ccagcctggc 1920
   ctctaggcct ggaagctgtg gctctctggc ctaacacagc tcctcgaagc ctcatacgca 1980
   tacctcatca ggcagaactg gggaattgaa gttacataga caaaggcctt gaggccactg 2040
   tacctaagcc gagcatggta gttaggcaag aactatgaac aggtcttctg aagtgactgc 2100
   cactagttct gacttgggga atatggtccc ttaaacatgc caaagtgagc tttttaagtg 2160
   gataagttag agatttctct ccccagatga aacgtgaagt atagcttgtt gggttttggc 2220
   ttgttgccta taggctctta aggtacttta gacaagagag gaaggtgtct taggtgctgt 2280
   catagactca ctcctgggtg acagcattca attctctatg gtggtaaaaa gctgattaga 2340
   gtttctgctg gtgggacttc cacttctact ggtcttttta tttcagaagt aataaagctc 2400
   tacatgcaac ttgcaaaaaa aaaaaaaaaa aaaaaaaaaa aaaaaaaaaa aaaaaaaaaa 2460
   aaaaaaaaaa aaaaaaaaaa aaa 2483